# EUROPEAN PATENT APPLICATION

(11) **EP 4 215 545 A1**
(43) Date of publication of application: **26.07.2023**
(21) Application number: 21869483.4
(22) Date of filing: 16.09.2021
(51) Int. Cl.: C07K 16/10, A61K 39/395, A61P 31/14, C07K 16/46, C12N 15/13, G01N 33/53, G01N 33/569

(54) **ANTIBODY AGAINST CORONAVIRUS**

(30) Priority: 16.09.2020 JP 2020155314; 19.02.2021 JP 2021025438
(71) Applicant: National University Corporation Tottori University, Tottori-shi, Tottori 680-8550 (JP)
(72) Inventor: TAKAHASHI, Yoshimasa, Tokyo 162-8640 (JP); ONODERA, Taishi, Tokyo 162-8640 (JP); ADACHI, Yu, Tokyo 162-8640 (JP); OSHIMURA, Mitsuo, Yonago-shi, Tottori 683-8503 (JP); KAZUKI, Yasuhiro, Yonago-shi, Tottori 683-8503 (JP); SATOFUKA, Hiroyuki, Yonago-shi, Tottori 683-8503 (JP); HASHIGUCHI, Takao, Kyoto-shi, Kyoto 606-8501 (JP); MAENAKA, Katsumi, Sapporo-shi, Hokkaido 060-0808 (JP)
(74) Representative: J A Kemp LLP
(86) International application number: PCT/JP2021/035212
(87) International publication number: WO 2022/059801

(57) **Abstract**

Provided is a human antibody against a spike protein of coronavirus, in particular, against a protein in an extracellular region or a receptor-binding domain.

## Description

### Technical Field

The present invention relates to an antibody against coronavirus, which is produced using human antibody-producing mice.

### Background Art

Antibodies have been used as therapeutic drugs for cancer, rheumatoid arthritis, etc. in the medical field. For example, Trastuzumab has been used as a molecular target antibody drug against HER2 (or ErbB2) on the surface of cancer cells for the treatment of breast cancer. On the other hand, Tocilizumab is a humanized anti-IL-6 receptor antibody, which has been used as a therapeutic drug for rheumatoid arthritis.

In order to enhance the therapeutic effects and safety of an antibody when it is administered to a human, the antibody is desirably a human antibody. The present inventor had previously produced a mouse artificial chromosome (MAC) vector (Japanese Patent No. 5557217 (Patent Literature 1) and WO2019/177163 (Patent Literature 2)). The present inventor had incorporated a human antibody heavy chain gene and a human antibody light chain gene (a κ gene and/or a λ gene) into the produced vector, and had then allowed a mouse to retain the thus prepared vector, so that the present inventor had successfully produced a complete human antibody-producing mouse (WO2018/079857 (Patent Literature 3)).

Meanwhile, severe acute respiratory syndrome coronavirus 2 (SARS-CoV2) is an emerging coronavirus that has caused pandemic (global pandemic) due to currently ongoing novel coronavirus disease (COVID-19). SARS-CoV2 has caused a large number of infected people and dead people over the world. In order to stop the spreading of the pandemic due to this zoonotic virus, it becomes top priority to discover vaccines or therapeutic drugs therefor.

Since a SARS-CoV-2 spike (S) glycoprotein promotes the invasion of the virus into host cells, this glycoprotein is a main target of a neutralizing antibody. There are several reports regarding a monoclonal antibody that targets the S glycoprotein of SARS-CoV2 (Wang1 C. et al., NATURE COMMUNICATIONS (2020) 11: 2251 (Non Patent Literature 1); Pinto D. et al., Nature, Vol. 583, 290-295, 2020 (Non Patent Literature 2)).

Moreover, it has also been reported that an antibody having neutralizing activity has been obtained from patients who had recovered from novel coronavirus infection (Wu Y. et al., Science 10.1126, 2020 (Non Patent Literature 3)).

### Citation List

### Patent Literature

Patent Literature 1: Japanese Patent No. 5557217
Patent Literature 2: WO2019/177163
Patent Literature 3: WO2018/079857

### Non Patent Literature

Non Patent Literature 1: Wang1 C. et al., NATURE COMMUNICATIONS (2020) 11: 2251
Non Patent Literature 2: Pinto D. et al., Nature, Vol. 583, 290-295, 2020
Non Patent Literature 3: Wu Y. et al., Science 10.1126, 2020

### Summary of Invention

### Technical Problem

However, it has been desired to develop an antibody, which can be applied to humans more safely than the above-described known antibodies against coronavirus, and further, is excellent in terms of affinity, neutralizing activity, blood kinetics, production costs, etc.

The present inventor has conducted intensive studies directed towards achieving the aforementioned object, and as a result, the present inventor has succeeded in obtaining an antibody having desired properties by using a human antibody-producing mouse, into which an artificial chromosome vector comprising human antibody genes has been introduced, thereby completing the present invention.

### Solution to Problem

Specifically, the present invention is as follows.
(1) An antibody against a spike protein of coronavirus.
(2) The antibody according to the above (1), wherein the spike protein is a protein in an extracellular region or a receptor-binding domain.
(3) The antibody according to the above (1) or (2), wherein the epitope of the spike protein is composed of at least two amino acid residues selected from the group consisting of R403, D405, E406, R408, T415, G416, K417, D420, Y449, Y453, L455, F456, G485, Y486, N487, Y489, Q493, S494, Y495, G496, Q498, N501, G502, V503, G504 and Y505, in the full-length amino acid sequence of the receptor-binding domain.
(4) The antibody according to the above (3), wherein the two amino acid residues are R403 and K417.
(5) The antibody according to the above (1), wherein the coronavirus is SARS-CoV1, SARS-CoV2, or MERS-CoV.
(6) The antibody according to any one of the above (1) to (5), which has a neutralizing activity of less than 1 ng/ml as an IC50 value that is an indicator of suppression of viral proliferation.
(7) The antibody according to any one of the above (1) to (6), wherein the amino acid sequence of a heavy chain variable region thereof is as set forth in SEQ ID NO: 14 or 18.
(8) The antibody according to any one of the above (1) to (6), wherein the amino acid sequence of a light chain variable region thereof is as set forth in SEQ ID NO: 16 or 20.
(9) An antibody that binds to an epitope, to which the antibody according to any one of the above (1) to (8) binds.
(10) The antibody according to any one of the above (1) to (9), which is a chimeric antibody, a humanized antibody, or a human antibody.
(11) A pharmaceutical composition comprising the antibody according to any one of the above (1) to (10).
(12) The pharmaceutical composition according to the above (11), which is for use in the treatment of coronavirus disease.
(13) A reagent for detecting coronavirus, comprising the antibody according to any one of the above (1) to (10).
(14) A method for detecting coronavirus, comprising a step of allowing the antibody according to any one of the above (1) to (10) to come into contact with a collected biological sample.

### Advantageous Effects of Invention

According to the present invention, a human antibody against coronavirus is provided. The antibody of the present invention is a complete human antibody obtained by immunizing a complete human antibody-producing mouse with a S protein of coronavirus, and the present antibody is excellent in terms of affinity and neutralizing activity and is useful for the treatment or prophylaxis of coronavirus disease, etc.

### Brief Description of Drawings

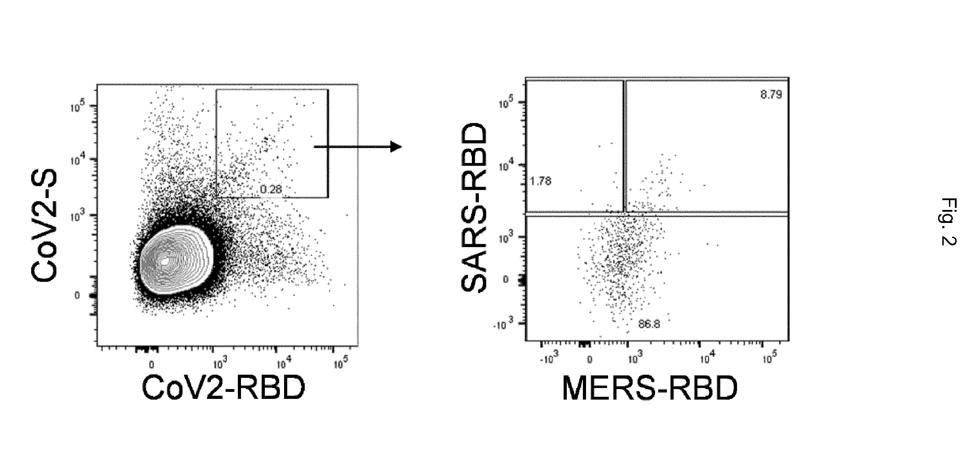

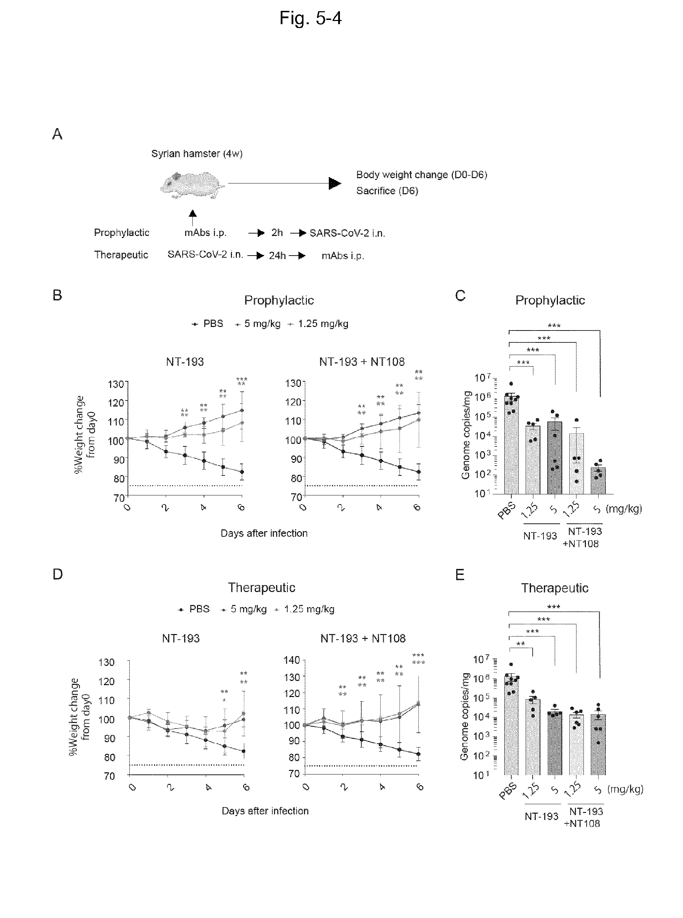

[Figure 1] Figure 1 is a view showing a schedule of immunization of human antibody-producing mice with RBD.
[Figure 2] Figure 2 is a view showing the results of separation of memory B cells.
[Figure 3] Figure 3 is a view showing the results of isolation of antibodies against individual coronaviruses.
[Figure 4] Figure 4 is a view showing the results obtained by measuring the neutralizing activities of antibodies, using pseudoviruses.
[Figure 5-1] Figure 5-1 is a view showing that two RBS antibody clones exhibit different cross-neutralizing activities against SARS.
   (A) CoV2 S-binding monoclonal antibodies were established from immune mice expressing only human antibodies according to a single cell culture approach. S and RBD proteins directed to bind to the monoclonal antibodies in the culture solution were evaluated according to an ELISA method. Evaluation was carried out using a CoV2 neutralizing activity pseudovirus system in the culture solution. The two clones (#108: NT-108; and #193: NT-193) exhibited neutralizing activity.
   (B) The amount of CoV2 RBD that bound to a hACE-coating plate was evaluated after pre-incubation with serially diluted NT-108/NT-193. Antibody-mediated inhibition against the hACE2-RBD binding was plotted as a percentage.
   (C, D) The neutralizing activities of the antibodies against CoV2 were evaluated using pseudovirus (C) and authentic virus (D). IC50 is shown in the upper portion of each figure.
   (E) An RBD-binding plate was masked with a competitive antibody, and NT-108 and NT-193 were then loaded thereon. The binding of the antibodies to unmasked RBD was evaluated.
   (F) The binding affinity of NT-108 and NT-193 to CoV2 RBD is evaluated by an SPR analysis.
   (G) The neutralizing activities of the antibodies against CoV1 were evaluated using pseudovirus.

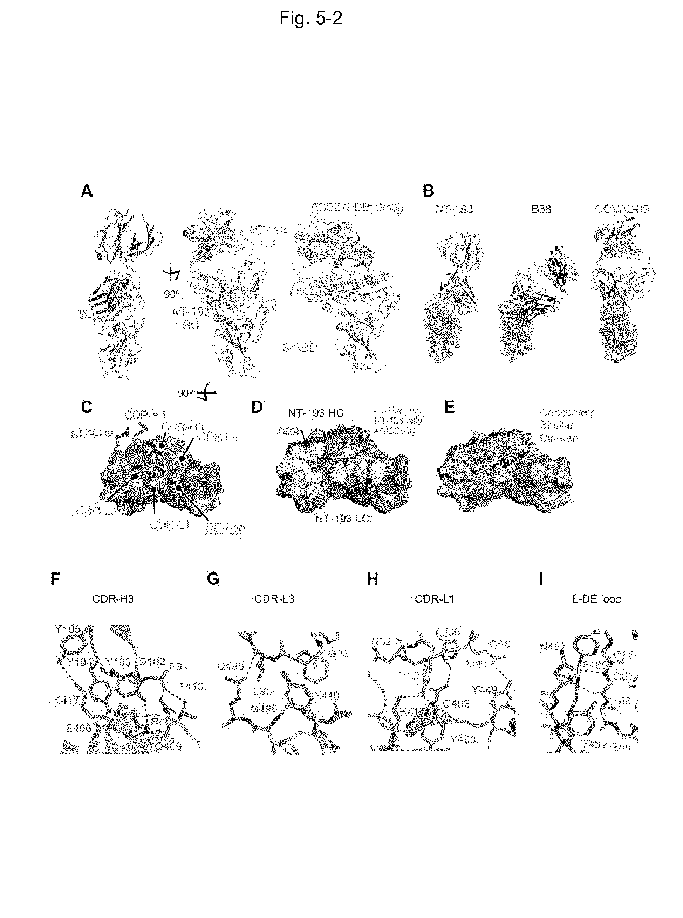

[Figure 5-2] Figure 5-2 is a view showing the structure of an NT-193 Fab-S-RBD complex.
   (A) Left - Models of outline pictures of an NT-193 complex obtained at two angles (reddish purple: NT-193 heavy chain; pink: NT-193 light chain; orange: S-RBD; and Nos. 1 to 3 indicate CDR-1, 2, and 3, respectively). Right - The structure of an ACE2-S-RBC complex (PDB: 6m0j), in which the same angles as those for the NT-193 complex (right) are applied. ACE2 is shown with light blue.
   (B) Comparison of the NT-193 complex (colored with the same color as that in A) with representative class 1 antibody complexes, CC12.1 (dark blue and gray, RBS-A subclass) and COVA2-39 (dark and light brown, RBS-B subclass) subclasses. The left model is shown with the same angle as one NT-193 complex on the right side in (A). The left and right angles are rotated at an angle of almost 90°.
   (C) CDR-binding sites (picture model; reddish purple color and pink color) are shown on a surface model of the receptor-binding site (RBS) of S-RBD (gray). The angles of the RBSs shown in (C) to (E) are the same.
   (D) Comparison between ACE and NT-193 binding sites on RBS. ACE only, NT-193 only, and the ACE and NT-193 (overlapping) binding site are shown with light blue, reddish purple, and yellow, respectively.
   (E) Preservability of the sequence numbers of RBSs between SARS-CoV-1 and SARS-CoV-2. Preserved: reddish purple; similar: pink; and not preserved: gray.
   (F to I) Detailed recognition modes of: CDR-H3 of NT-193 (F), CDR-L1 (G), DE loop of L chain (H), and CDR-H3(I). The same coloration as that in (A) is applied. The dotted line indicates polar interaction.

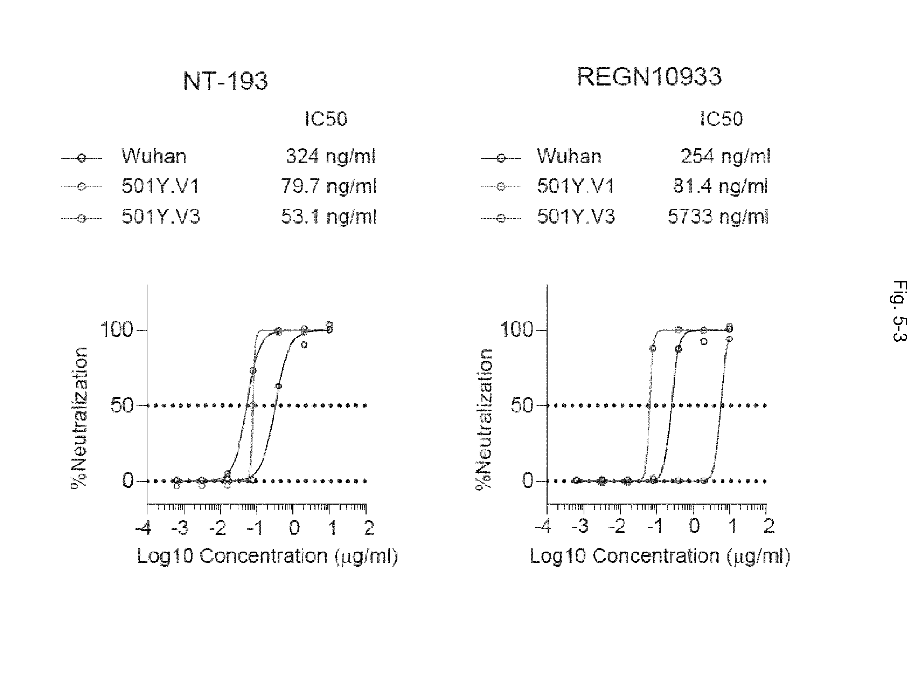

[Figure 5-3] Figure 5-3 is a view showing that NT-193 exhibits neutralizing activity also against variant strains.
   (A) The neutralizing activity of the antibody against 501Y.V1 and 501Y.V3 viruses was compared with that against the original virus (WK-521). [Figure 5-4] Figure 5-4 is a view showing the prophylactic and therapeutic effects of NT-193 and an NT-193 + NT-108 cocktail.
   (A) A schematic view of an experimental design for evaluating the prophylactic effects and therapeutic effects of NT-193 and an NT-193 + NT108 cocktail.
   (B, D) The body weights of CoV2-loaded hamsters, which were administered with either prophylactic (B) or therapeutic (D) mAb at an indicated dose were monitored on a daily basis.
   (C, E) The loaded amount of CoV2 virus in the lung tissues of hamsters, which were administered with either prophylactic (B) or therapeutic (D) mAb at an indicated dose.

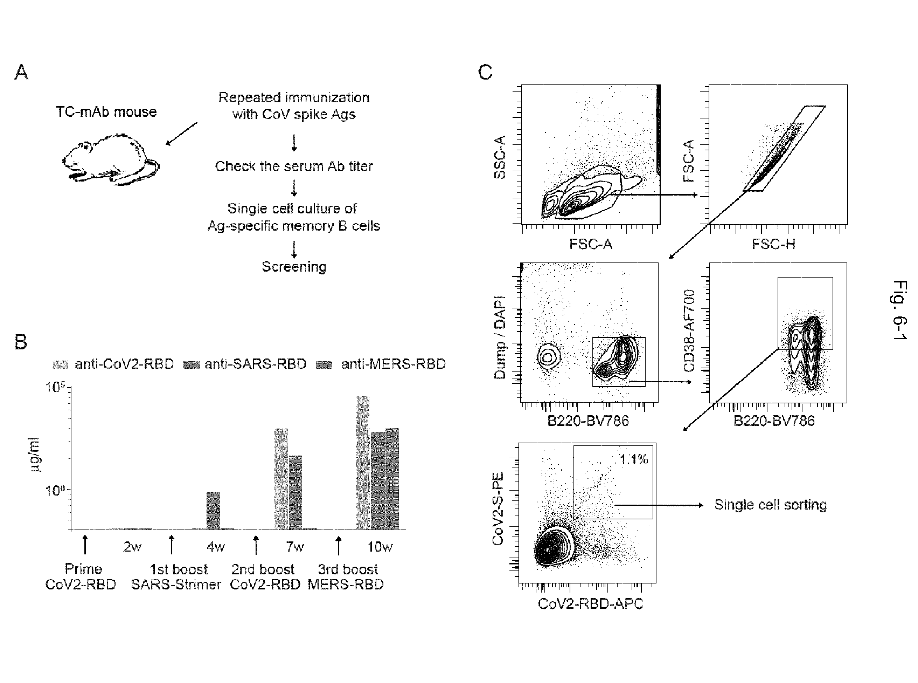

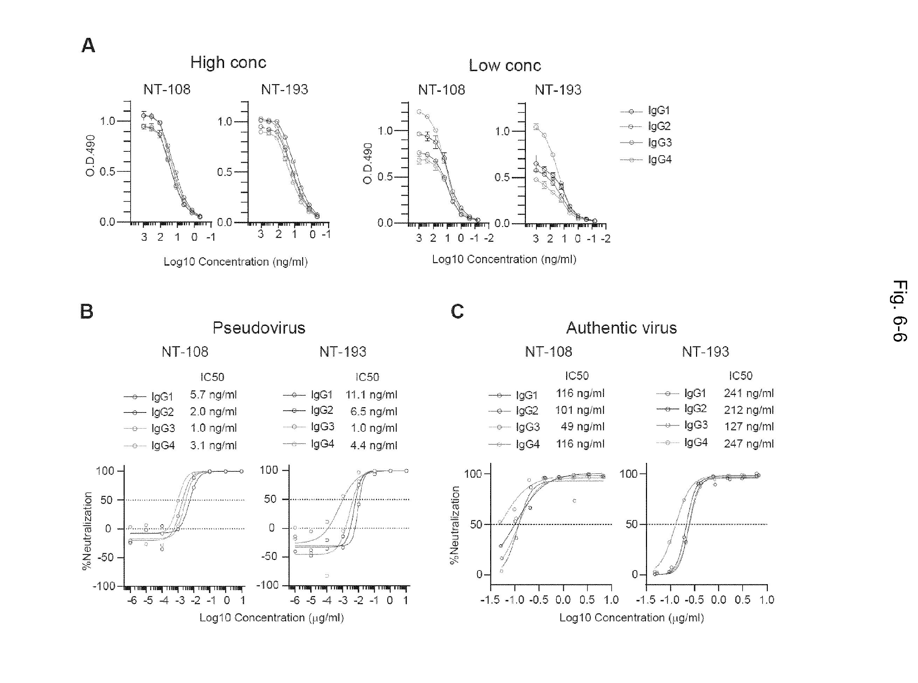

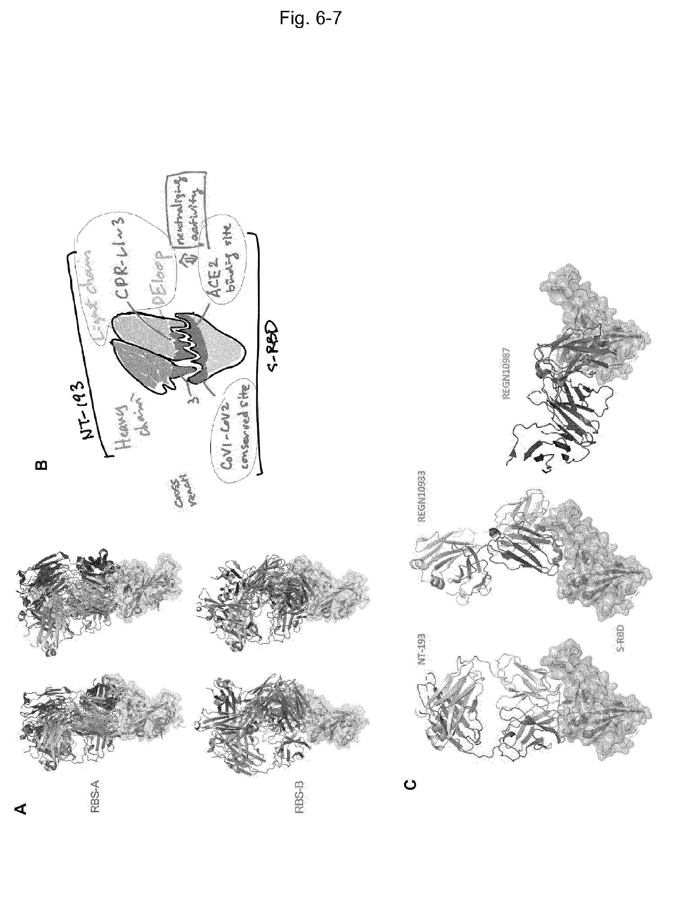

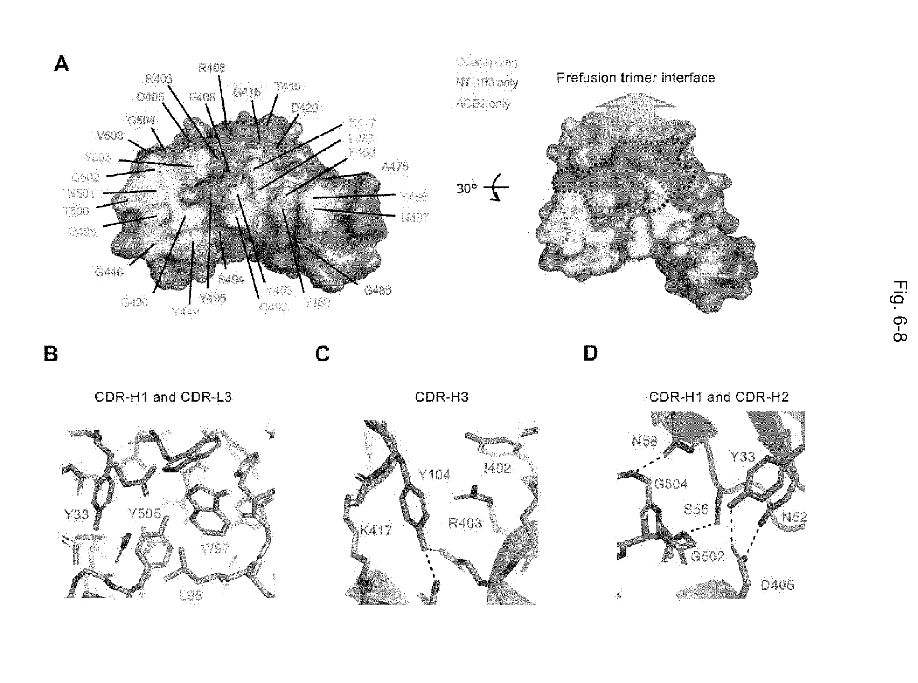

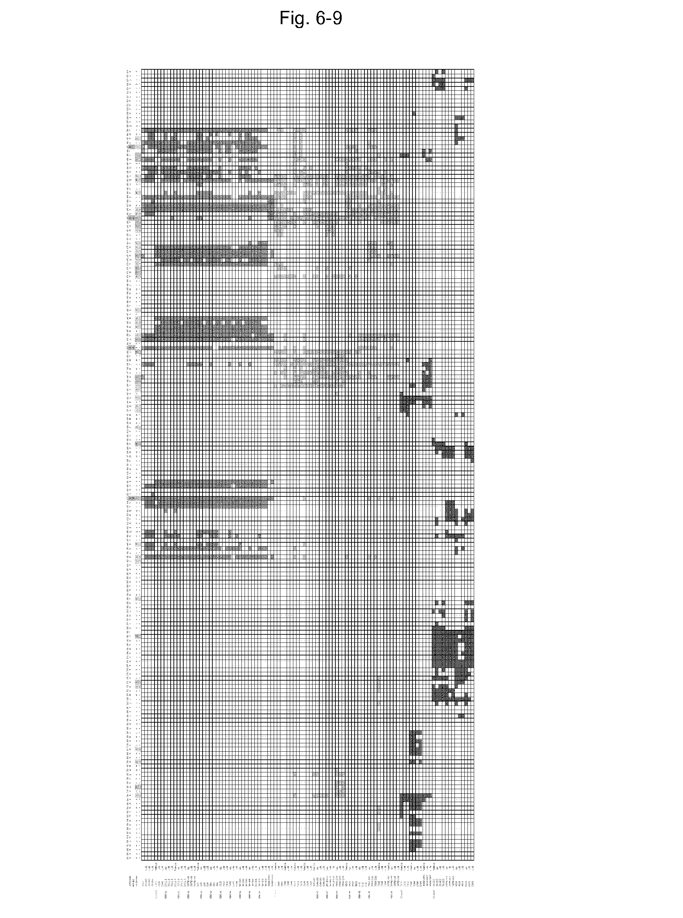

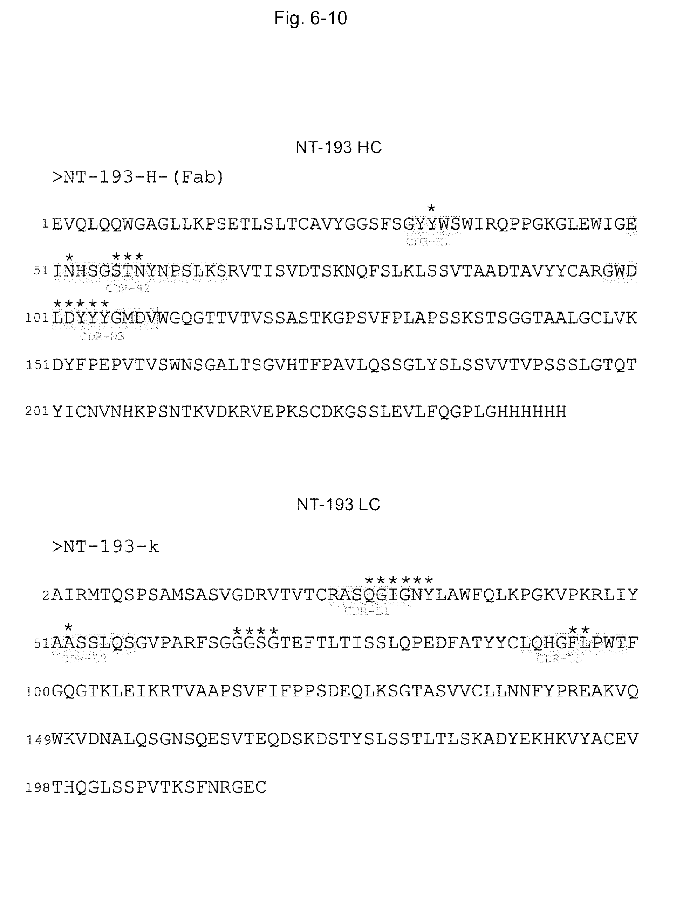

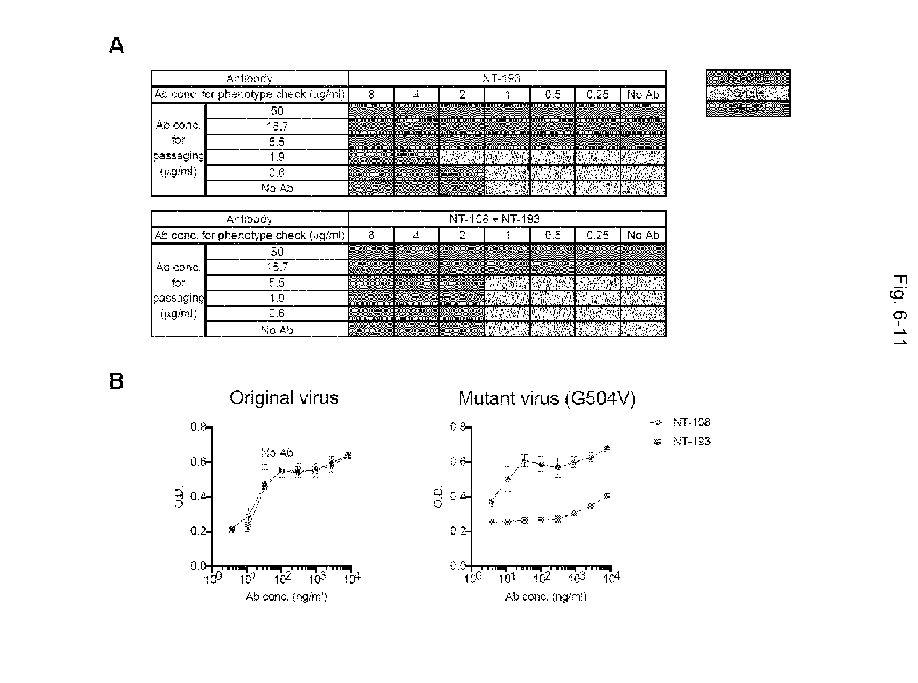

[Figure 6-1] Figure 6-1 is a view showing a method of isolating a CoV2 RBD-binding monoclonal antibody (mAb) by the single cell culture of memory B cells, and an isolation process.
   (A) A schematic view of an experimental work flow for isolating RBD mAb from a TC-mAb mouse.
   (B) The antibody titers of serum human IgG against RBDs derived from CoV2 (red), SARS (blue) and MERS (gray) were evaluated after priming with several coronavirus antigens and continuous boosting, as shown in the figure.
   (C) Single cell sorting of CoV2 RBD-binding memory B cells
      Class-switched memory B cells were concentrated from splenic cells according to a MACS system, and were then stained with fluorescent Ab and probes. The frequency of CoV2 S/RBD-binding memory B cells in the class-switched memory B cells was as shown in the figure.

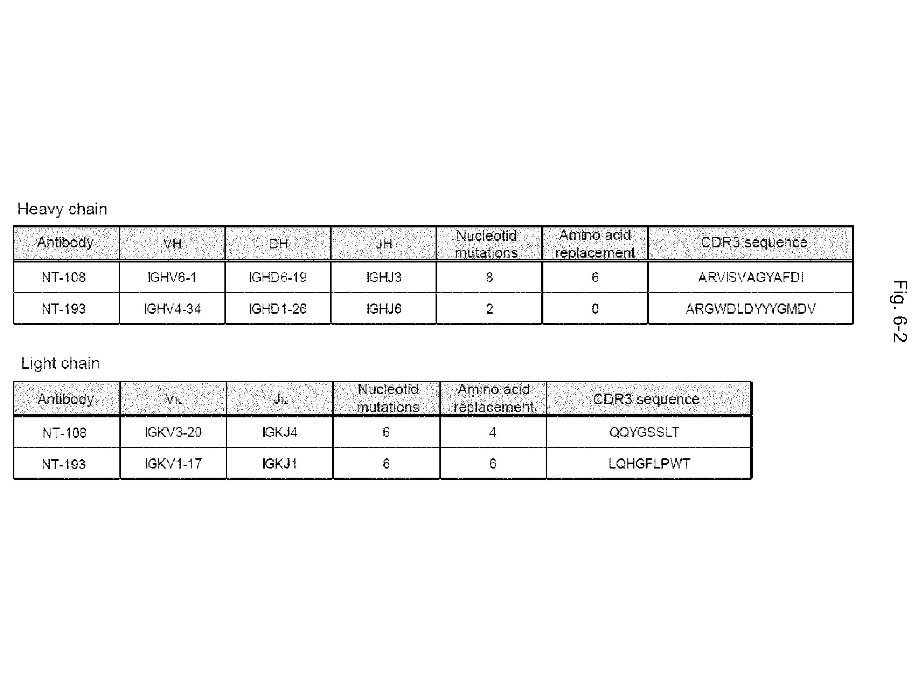

[Figure 6-2] Figure 6-2 is a view showing the use amounts, mutations, and clone types of the heavy chain variable region (VH) / light chain variable region (VL) of NT-108 and NT-193.
   The heavy chain is shown in the upper view, whereas the light chain is shown in the lower view.

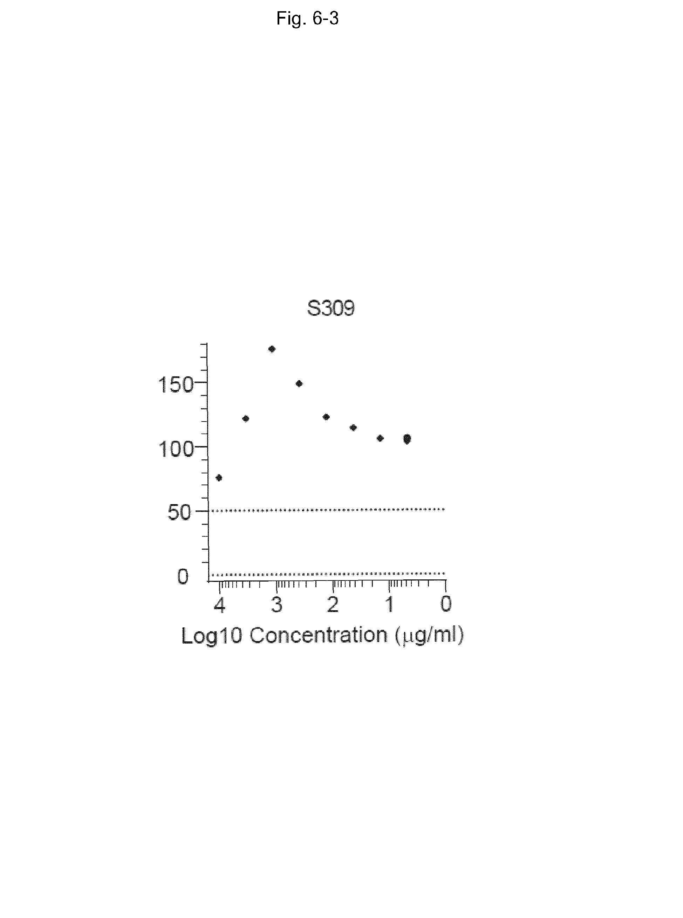

[Figure 6-3] Figure 6-3 is a view showing inhibition of hACE2 binding by S309 mAb.
   After pre-incubation with serially diluted S309 (class 3) mAb, the amount of CoV2 RBD that bound to an hACE-coating plate was evaluated. Antibody-mediated inhibition against the hACE2-RBD binding was plotted as a percentage.

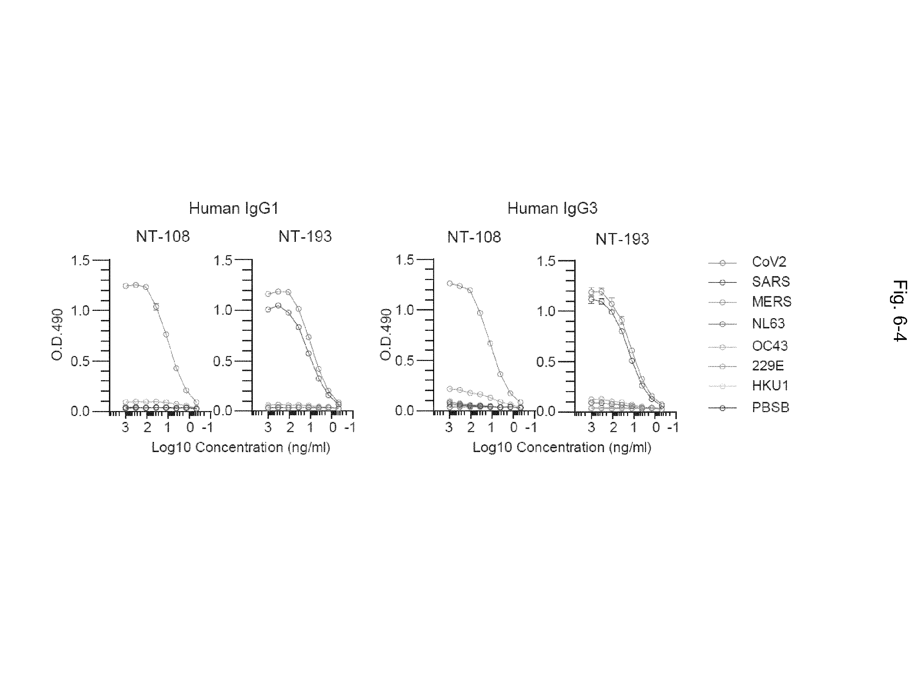

[Figure 6-4] Figure 6-4 is a view showing the cross-reactivity of NT-108 and NT-193 mAb to several coronavirus S-trimeric antigens.
   Several coronavirus-derived S-trimeric antigens were coated on an ELISA plate, and the binding of serially diluted NT-108 and NT-193 thereto was then evaluated. The left view shows human IgG1 type, whereas the right view shows a human IgG3 type.

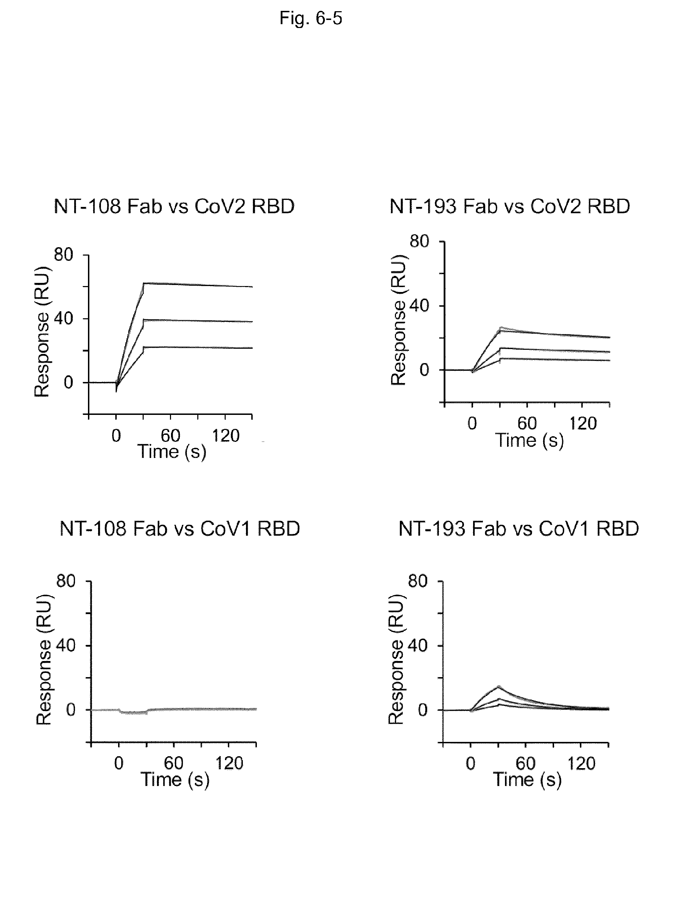

[Figure 6-5] Figure 6-5 is a view showing the results of the SPR analysis of the binding of NT-108 and NT-193 Fab to SARS-CoV RBD.
   The binding of the serially diluted Fab fragment of NT-108 or NT-193 to immobilized SARSCoV-1 RBD or SARS-CoV-2 RBD was evaluated by an SPR analysis.
[Figure 6-6] Figure 6-6 is a view showing that NT-108 and NT-193 increase neutralizing activity, in relation to IgG3 subclass.
   (A) An ELISA plate was coated with low-concentration (0.2 µg/ml) and high-concentration (5 µg/ml) CoV2 S-trimers. The amounts of NT-108 and NT-193 that bound to the S-trimers of individual IgG subclasses were plotted with O.D.
   (B, C) The neutralizing activities of the antibodies against CoV2 virus were measured using pseudovirus (B) and authentic virus (C). IC50 is shown in the upper portion of each figure.
[Figure 6-7] Figure 6-7 is a view showing comparisons regarding the structures of S-RBD complexes.
   (A) Models of comparative pictures of an NT-193 complex with class 1 antibody complexes, RBS-A (dark blue and gray) and RBS-B (dark and light brown) subclasses (reddish purple: NT-193 heavy chain; pink: NT-193 light chain; orange: S-RBD). The left model is shown at the same angle as the right one of the NT-193 complexes shown in Figure 2(A). The left and right models are rotated at an angle of almost 90°.
   (B) The positions of the CDRs and DE loop of NT193 on S-RBD.
   (C) An outline of NT-193 (left, with the same color as that in (A)), REGN10933 (center) and REGN10987 (right), with an S-RBD complex. The schematic view was made by hand (green: REGN10933; blue: REGN10987).
[Figure 6-8] Figure 6-8 is a view showing the binding sites of an NT-193 Fab-S-RBD complex.
   (A) Comparison between the ACE-binding site and the NT-193-binding site on RBS. ACE only, NT-193 only, and the ACE and NT-193 (overlapping) binding site are shown with light blue, reddish purple, and yellow, respectively.
   (B to D) Stick models for specifically showing the CDR-binding sites of CDR-H1/L3 (B), CDR-H3 (C), and CDR-H1/H2 (D) (reddish purple: NT-193 heavy chain; pink: NT-193 light chain; orange: S-RBD). The dotted line indicates polar interaction.
[Figure 6-9] Figure 6-9 is a view showing a mapping of the antibody-binding residues of S-RBD.
   The binding residues of the S-RBD of each antibody are shown by color coding. The SARS-CoV-2 residue that is not preserved in CoV-1 is shown with yellow. The reported mutation (pink) is shown in the column between the sequences of SARS-CoV-1 and CoV-2.
[Figure 6-10] Figure 6-10 is a view showing the amino acid sequences of the heavy chain (HC) and light chain (LC) of NT-193.
   The binding residues are shown with asterisks. It shows a secondary structure. [Figure 6-11] Figure 6-11 is a view showing the screening of NT-193 for escape mutants.
   (A) SARS-CoV-2 virus was sub-cultured three times in the presence of a designated antibody, and thereafter, it was verified regarding escape. Viral RNA was recovered from wells using CPE and was sequenced. Mutant amino acid residues in RBD are shown.
   (B) The neutralizing activities of the antibodies against the mutant virus were evaluated.

### Description of Embodiments

### 1. Outline

The present invention relates to an antibody against coronavirus.

The present inventor has focused on a spike protein of coronavirus, and has successfully produced a human antibody against the spike protein by using a complete human antibody-producing mouse, into which human antibody genes have been introduced.

The coronavirus used as a target in the present invention includes a severe acute respiratory syndrome virus (referred to as "SARS-CoV 1" or "SARS-CoV"), a middle east respiratory syndrome virus (referred to as "MERS-CoV"), and a novel severe acute respiratory syndrome virus (referred to as "SARS-CoV2" or "SARS-CoV-2").

The present inventor has conducted intensive studies regarding the neutralizing activity, affinity, and cross-reactivity of the above-described antibody, and as a result, the present inventor has found that the neutralizing activity of the above-described antibody against SARS-CoV2 is significant, compared with those of the existing antibodies. Moreover, the present inventor has discovered an antibody binding to SARS-CoV1, MERS-CoV and SARS-CoV2, in addition to a clone specific to SARS-CoV2 and a clone exhibiting cross-reactivity to SARS-CoV1 and SARS-CoV2.

The antibody of the present invention binds to the extracellular region or the receptor-binding domain (RBD) of a spike protein. Even in an initial stage of coronavirus infection, the antibody of the present invention can detect living viruses existing in body fluids such as blood, saliva and nasal mucus, with high sensitivity. Thereby, it is possible to examine the presence or absence of infection with coronavirus (i.e. positive or not).

### 2. Antibody against coronavirus

### (1) Antigenic protein

The proteins used as antigens for producing the antibody of the present invention herein are proteins in the extracellular region and the receptor-binding domain (RBD) of a spike protein of coronavirus (S protein). Such proteins can be prepared by a genetic engineering technique.

The viral protein may be a native protein purified from the tissues or cells of a mouse, a rat, a human and the like, or may also be a protein produced by a genetic engineering technique.

In addition, the method for producing the protein (polypeptide) used as an antigen may be either a chemical synthesis or a synthesis by a genetic engineering technique using *Escherichia coli,* etc. A method well known to those skilled in the art can be used.

In the case of chemically synthesizing a polypeptide, the polypeptide can be synthesized according to a publicly known peptide-synthesizing method. Moreover, for such synthesis, both of a solid phase synthesis method and a liquid phase synthesis method can be applied. A commercially available peptide-synthesizing device (for example, PSSM-8, etc.; manufactured by Shimadzu Corporation) may also be used.

In the case of synthesizing a polypeptide according to a genetic engineering technique, first, DNA encoding the polypeptide is designed and synthesized, and a PCR method can be carried out using primers designed to amplify a desired DNA region. Then, the above-described DNA is ligated to a suitable vector to obtain a recombinant vector for protein expression. This recombinant vector is introduced into a host such that a gene of interest can be expressed therein, thereby obtaining a transformant (Sambrook J. et al., Molecular Cloning, A Laboratory Manual (4th edition) (Cold Spring Harbor Laboratory Press (2012)).

For example, nucleotide sequences encoding the extracellular region and RBD region proteins of the S proteins of SARS-CoV2, SARS-CoV1 and MERS-CoV are artificially synthesized. These nucleotide sequences are each inserted into a vector, into which a tag for protein purification (e.g. His tag) has been inserted, as necessary, so as to produce an expression vector.

The host used in transformation is not particularly limited, as long as it can express a gene of interest. Examples of such a host may include bacteria *(Escherichia coli, Bacillus subtilis,* etc.), yeasts, animal cells (COS cells, CHO cells, etc.), insect cells, and insects. Mammals such as goats can also be used as such hosts. The method of introducing a recombinant vector into a host is known (Sambrook J. et al., Molecular Cloning, A Laboratory Manual (4th edition) (Cold Spring Harbor Laboratory Press (2012)).

The above-described expression vector is introduced into predetermined cells for protein expression, and a recombinant protein is then purified from a culture supernatant. Regarding the purification, common biochemical methods used in isolation and purification of proteins, such as, for example, ammonium sulfate precipitation, gel filtration, ion exchange chromatography and affinity chromatography, are applied alone or by combining these methods, as appropriate, so that an antigenic protein of interest can be isolated and purified.

Besides, in the present invention, an antigenic protein can also be obtained by *in vitro* translation using a commercially available cell-free synthetic system, for example, Expressway^{™} System.

In the present invention, the protein used as an antigen is not limited, and it may be either a full length, or at least a portion of each of the amino acid sequences as set forth in the following sequence numbers. The protein used herein as an antigen is preferably at least a portion of the extracellular region, or the receptor-binding domain.

In the present invention, the amino acid sequences of the spike proteins of individual coronaviruses, and the nucleotide sequences of DNAs encoding the proteins, are shown in Table 1 below.

**[Table 1]**

| Virus | Antigen | Nucleotide sequence | Amino acid sequence |
|---|---|---|---|
| SARS-CoV2 | Full length | SEQ ID NO: 1 | SEQ ID NO: 2 |
| | RBD | SEQ ID NO: 3 | SEQ ID NO: 4 |
| SARS-CoV 1 | Full length | SEQ ID NO: 5 | SEQ ID NO: 6 |
| | RBD | SEQ ID NO: 7 | SEQ ID NO: 8 |
| MERS-CoV | Full length | SEQ ID NO: 9 | SEQ ID NO: 10 |
| | RBD | SEQ ID NO: 11 | SEQ ID NO: 12 |

Moreover, in the present invention, the antigens may include: (a) proteins (polypeptides) comprising the amino acid sequences as set forth in the above-described sequence numbers; (b) proteins each comprising an amino acid sequence comprising a deletion, substitution or addition of one or several amino acids (for example, 1 to 10, 1 to 5, 1 to 3, 1 or 2 amino acids, etc.) with respect to the amino acid sequences as set forth in the above-described sequence numbers; and (c) proteins each comprising an amino acid sequence having an identity of 70% or more to the amino acid sequences as set forth in the above-described sequence numbers.

### (2) Immune animals

Upon the production of antibodies in the present invention, the animals used as targets of immunization are not particularly limited, and mice, rats, rabbits, goats, horses, sheep and the like, which are generally used in the production of antibodies, can be used. The immune animals are preferably complete human antibody-producing mice (TC mice).

The present inventor has succeeded in producing a mouse that retains a full-length human Ig gene locus and produces a complete human antibody, according to a trans chromosomic (TC) mouse production technique, for the first time over the world (Tomizuka, K.et al., Nature Genetics, (1997)16, 133-143; Tomizuka, K.et.al., Proc. Natl. Acad. Sci. USA, (2000)97, 722-727). The artificial chromosome vector used herein is derived from a human, but as a result of the subsequent studies, the present inventor has succeeded in producing TC mice, using a mouse artificial chromosome vector (MAC) (WO2018/079857).

Herein, the mouse artificial chromosome vector means a mini chromosome consisting only of a telomere, a centromere and a replication origin, wherein the mini chromosome is shortened by removing unnecessary genes from a native mouse chromosome. Into this mouse artificial chromosome vector, a loxP sequence that is a cloning site can be introduced. The mouse artificial chromosome vector is advantageous in that (i) it can comprise any given novel gene, (ii) it can be independently maintained without being inserted into a host chromosome, and can transmit to off-springs, (iii) it is stably retained in mammalian cells including mice or rats at a constant copy number for a long period of time, (iv) the length of an introducible DNA is not limited, and the like.

For incorporation of a gene of interest (for example, a human antibody gene) into the mouse artificial chromosome vector, two cloning methods, namely, an annular insertion-type cloning method and a chromosomal translocation-type cloning method can be utilized (Reference: Uno N, Abe S, Oshimura M, Kazuki Y. J Hum Genet. 2018 Feb;63(2):145-156. doi: 10.1038/s10038-017-0378-7.).

Since it has been known that ES cells and iPS cells contribute to germ lines, these cells, into which a mouse artificial chromosome vector comprising a human antibody gene of interest has been introduced, are injected into the blastocyst of a mouse embryo. Thereafter, the thus obtained embryo is transplanted into the uterus of a foster mother, and the mother is then allowed to give birth, so as to produce trans chromosomic (TC) mice. Further, the thus obtained male and female TC mice are mated with each other to produce off-spring mice (these mice are referred to as "TC mice") (WO2018/079857).

In one embodiment of the present invention, the above-described TC mouse is used as an immunization target.

With regard to human Ig heavy chain and light chain (κ and/or λ), a complete human antibody-producing mouse can be produced by introducing the above-described MAC vector into a mouse (referred to as a "TC mouse") and then destroying the endogenous mouse Ig gene group of the TC mouse.

The human antibody-producing mouse can be obtained according to the above-described known method, but it can also be obtained as a commercially available product (TC-mAb^{™} Mouse: Trans Chromosomics, Inc.).

### (3) Immunization of mice with virus antigen

In the present invention, animals are immunized by administration with the produced antigen. In the present invention, for example, a complete human antibody-producing mouse (TC mouse) can be used as an animal to be immunized.

The antigenic protein produced as described above is administered per se or together with a carrier and a diluent to an animal (for example, a TC mouse), so that the animal is immunized. The applied dose of the antigen per animal is 1 µg to 10 mg, when an adjuvant is used. Examples of the adjuvant used herein may include a Freund's complete adjuvant (FCA), a Freund's incomplete adjuvant (FIA), and an aluminum hydroxide adjuvant. Immunization is mainly carried out by injecting the antigenic protein into the vein, subcutis, abdominal cavity, or the like. In addition, intervals of immunization are not particularly limited, and immunization is carried out 2 to 20 times with intervals of several days to several weeks, and preferably with intervals of 1 or 2 weeks.

### (4) Antibody isolation

Blood is collected from an immunized animal (for example, a TC mouse), and the antibody titers of blood IgG antibodies against the S proteins of individual viruses (an extracellular region and an RBD region) are measured by sandwich ELISA, and the splenic cells of the mouse, in which an IgG antibody has been induced, are collected. The collected splenic cells are treated with an anti-FcyRII/III antibody, and are then stained with the antibodies described below and S protein fluorescent probes, so that memory B cells that bind to the S protein fluorescent probes are fractionated by flow cytometry. The fractionated memory B cells are cultured, and then, on Day 10 after the culture, a culture supernatant is recovered. Upon the culture, the memory B cells are seeded, in an amount of 1 cell/well, on feeder cells in which suitable mouse CD40L and BAFF are expressed. As a medium, an RPMI medium supplemented with various types of cytokines (for example, mouse IL-2, IL-4, and IL-5) can be used.

Moreover, in the present invention, it is also possible to produce a monoclonal antibody according to an ordinary hybridoma method.

In order to obtain hybridomas, cell fusion is carried out between antibody-producing cells and myeloma cells. The fusion operations can be carried out according to known methods, for example, the method of Kohler et al. As myeloma cells to be fused with antibody-producing cells, commonly available cells established from animals such as mice can be used. Examples of the myeloma cells may include mouse myeloma cell lines, such as P3X63-Ag8, P3X63-Ag8U.l, SP2/O-Ag14, PAI, P3U1, NSI/1-Ag4-1, and NSO/1.

Thereafter, hybridomas of interest are selected from cells after completion of the cell fusion treatment. As such a method of obtaining hybridomas, a cell suspension is appropriately diluted, and the cells are then dispersed on a microtiter plate in an amount of about 0.3 cells/well, as calculated according to a limiting dilution method. A selective medium such as a HAT medium is added to each well, and thereafter, a culture is carried out, while appropriately exchanging the selective medium with a fresh one. As a result, cells growing after initiation of the culture in the selective medium can be obtained as hybridomas.

### (5) Production of antibody protein according to genetic engineering technique

In the case of producing an antibody protein according to a genetic engineering technique, cDNA is synthesized from memory B cells that have been found to have binding ability to individual S proteins according to an ELISA method, and antibody genes (heavy chain and light chain V regions) are then cloned according to a PCR method. The cloned heavy chain V region is introduced into a mammalian expression vector comprising the C region of human IgG1 or IgG3, whereas the cloned light chain V region is introduced into a mammalian expression vector comprising the C region of human Igκ or Igλ, so as to produce individual antibody expression vectors. The thus produced antibody expression vectors are each introduced into host cells, and recombinant antibodies are then purified from the culture supernatant.

The antibody of the present invention may be a genetically recombinant antibody or an antigen-binding fragment, which is prepared and expressed according to a recombination means. For example, the antibody of the present invention may be a chimeric antibody, a humanized antibody, or a complete human antibody. The recombinant antibody of the present invention can be produced by the recombinant expression of a heavy chain and a light chain. For instance, in the case of a mouse-human chimeric antibody, antibody genes are isolated from mouse cells that produce antibodies against S proteins of viruses, and the heavy chain (H chain) constant region thereof is replaced with the H chain constant region gene of human IgG, which is then introduced into mouse myeloma cells, so as to obtain the mouse-human chimeric antibody. In the case of a humanized antibody, for example, the genes of the antigen-binding sites of an antibodies isolated from mouse cells that produce antibodies against S proteins are transplanted into human-derived antibody molecules, so as to produce the humanized antibody. Further, a human antibody can be produced by immunizing a mouse whose immune system has been replaced with that of a human, with an S protein.

The antibody protein obtained by the present invention is, for example, a complete human antibody, and is also a monoclonal antibody.

### (6) Production of antibody fragments

In the present invention, it is also possible to make fragments from the antibodies produced as described above.

The fragment of the antibody means a partial region of the antibody of the present invention, and examples of the antibody fragment may include Fab, Fab', F(ab')₂, Fv, diabody (dibodies), dsFv, and scFv (single chain Fv). The above-described antibody fragment can be obtained by cleaving the antibody of the present invention with various types of proteases, depending on purpose.

For instance, Fab can be obtained by treating an antibody molecule with papain, and F(ab')₂ can be obtained by treating an antibody molecule with pepsin. In addition, Fab' can be obtained by cleaving the disulfide bond of the hinge region of the above-described F(ab')₂.

In the case of scFv, cDNAs encoding the heavy chain variable region (H chain V region) and light chain variable region (L chain V region) of an antibody are obtained, and DNA encoding scFv is constructed. This DNA is inserted into an expression vector, and thereafter, the expression vector is introduced into a host organism and is expressed therein, so that scFv can be produced.

In the case of diabody, cDNAs encoding the H chain V region and L chain V region of an antibody are obtained, and DNA encoding scFv is constructed such that the length of the amino acid sequence of a peptide linker becomes 8 amino acid residues or less. This DNA is inserted into an expression vector, and thereafter, the expression vector is introduced into a host organism and is expressed therein, so that diabody can be produced.

In the case of dsFv, cDNAs encoding the H chain V region and L chain V region of an antibody are obtained, and DNA encoding dsFv is constructed. This DNA is inserted into an expression vector, and thereafter, the expression vector is introduced into a host organism and is expressed therein, so that dsFv can be produced.

The amino acid sequences of the heavy chain variable region and light chain variable region of the human antibody of the present invention, and the nucleotide sequences of DNAs encoding the concerned regions, are shown below.

### TC mice anti-CoV2 mAb VH sequences

NT-108 (NTCOV-1) VH (SEQ ID NO: 13) 
GAGGTGCAGCTGCAGCAGTCAGGTCCAGGACTGGTGAAGCCCTCGCAGACC
CTCTCACTCACCTGTTCCATCTCCGGGGACAGTGTCTCTAGCAACAGTGCTG
CTTGGAACTGGATCAGGCAGTCCCCATCGAGAGGCCTTGAGTGGCTGGGAA
GGACATACTACAGGTCCAAGTGGTATAATGATTATGCAGGAACTGTGAAAA
GTCGAATAGCCATCAACCCAGACACATCCAAGAACCAGTTCTCCCTGCACC
TGAACTCTGTGACTCCCGAGGACACGGCTGTGTATTTCTGTGCAAGAGTGAT
ATCAGTGGCTGGTTATGCTTTTGATATCTGGGGCCAAGGGACAATGGTCAC
CGTCTCCTCA
NT-108 (NTCOV-1) VH AA seq (SEQ ID NO: 14) 
QVQLQQSGPGLVKPSQTLSLTCSISGDSVSSNSAAWNWIRQSPSRGLEWLGRTY
YRSKWYNDYAGTVKSRIAINPDTSKNQFSLHLNSVTPEDTAVYFCARVISVAG
 YAFDIWGQGTMVTVSS
NT-108 (NTCOV-1) Vκ (SEQ ID NO: 15) 
GAAATTGTGTTGACACAGTCTCCAGGCACCCTGTCTTTGTCTCCAGGGGAAA
GAGCCACCCTCTCCTGCAGGGCCAGTCAGAGTGTTAGCAGCACCTTCTTAG
CCTGGTACCAGCAGAAACCTGGCCAGGCTCCCAGGCTCCTCATCTATGGTG
CATCCTACATGGCCACTGGCATCCCAGACAGGTTCAGTGGCAGTGGGTCTG
GGACAGACTTCACTCTCACCATCAGCAGACTGGAGCCTGAAGATTTTGCAG
TGTATTACTGTCAGCAGTATGGTAGCTCACTCACTTTCGGCGGGGGGACCA
AGCTGGAGATCAAA
NT-108 (NTCOV-1) Vκ amino acid sequence (SEQ ID NO: 16) 
EIVLTQSPGTLSLSPGERATLSCRASQSVSSTFLAWYQQKPGQAPRLLIYGASY
MATGIPDRFSGSGSGTDFTLTISRLEPEDFAVYYCQQYGSSLTFGGGTKLEIK
NT-193 (NTCOV-2) VH (SEQ ID NO: 17) 
GAGGTGCAGCTACAGCAGTGGGGCGCAGGACTGTTGAAGCCTTCGGAGACC
CTGTCCCTCACCTGCGCTGTCTATGGTGGGTCCTTCAGTGGTTACTACTGGA
GCTGGATCCGTCAGCCCCCAGGGAAGGGGCTGGAGTGGATTGGGGAAATC
AATCATAGTGGAAGCACCAACTACAACCCGTCCCTCAAGAGTCGAGTCACC
ATTTCAGTAGACACGTCCAAGAACCAGTTCTCCCTGAAGCTGAGCTCTGTG
ACCGCCGCGGACACGGCTGTGTATTACTGTGCGAGAGGTTGGGACCTAGAC
TACTACTACGGTATGGACGTCTGGGGCCAAGGGACCACGGTAACCGTCTCC
TCA
NT-193 (NTCOV-2) VH amino acid sequence (SEQ ID NO: 18) 
EVQLQQWGAGLLKPSETLSLTCAVYGGSFSGYYWSWIRQPPGKGLEWIGEINH
SGSTNYNPSLKSRVTISVDTSKNQFSLKLSSVTAADTAVYYCARGWDLDYYYG
MDVWGQGTTVTVSS
NT-193 (NTCOV-2) Vk (SEQ ID NO: 19) 
GACATCCGGATGACCCAGTCTCCATCTGCCATGTCTGCATCTGTAGGAGAC
AGAGTCACCGTCACTTGTCGGGCGAGTCAGGGCATTGGCAATTATTTAGCC
TGGTTTCAGCTGAAACCAGGGAAAGTCCCTAAGCGCCTGATCTATGCTGCA
TCCAGTTTGCAAAGTGGGGTCCCAGCAAGGTTCAGCGGCGGTGGATCTGGG
ACAGAATTCACTCTCACAATCAGCAGCCTGCAGCCTGAAGATTTTGCAACTT
ATTACTGTCTACAGCATGGTTTTCTCCCGTGGACGTTCGGCCAAGGGACCAA
GCTGGAGATCAAA
NT-193 (NTCOV-2) Vk amino acid sequence (SEQ ID NO: 20)

DIRMTQSPSAMSASVGDRVTVTCRASQGIGNYLAWFQLKPGKVPKRLIYAASS
LQSGVPARFSGGGSGTEFTLTISSLQPEDFATYYCLQHGFLPWTFGQGTKLEIK

### (7) Confirmation of binding ability of antibody according to ELISA

The binding activity of a monoclonal antibody against SARS-CoV2 RBD, SARS-CoV1 RBD, and MARS RBD is measured, for example, by an ELISA method.

### (8) Neutralization test

In the present invention, in order to evaluate the SARS-CoV2 virus infection-protecting effect of the antibody, a neutralization test can be carried out using VSV pseudovirus.

In the neutralization test, for example, an antibody (monoclonal antibody) that has been serially diluted with a medium is mixed with a viral S protein-expressing VSV pseudovirus that has been diluted with a medium, and the obtained mixture is then cultured.

By the way, TMPRSS2 (one type of Transmembrane protease, serine 2) has been known as a main protease that activates respiratory virus in the respiratory tract (Sakai K, et al., J Virol 88: 5608-5616, 2014; Makoto TAKEDA, Virus 69: 61-72, 2019). Also, Vero cells and Vero E6 cells, in which TMPRSS2 are constitutively expressed by a genetic engineering technique, have been known (i.e. Vero/TMPRSS2 cells and Vero E6/TMPRSS2 cells) (Shirogane Y, et al., J Virol 82: 8942-8946, 2008; Nao N, et al., PLoS One 14: e0215822, 2019). Recently, it has been clarified that these cell lines (in particular, Vero E6/TMPRSS2 cells) are extremely advantageous for separation and proliferation of SARS-CoV2 (Matsuyama S, et al., Proc Natl Acad Sci USA 117: 7001-7003, 2020).

Hence, in the present invention, after completion of the above-described culture, a mixed solution of the antibody and the virus is added to cells highly sensitive to coronavirus (for example, Vero E6/TMPRSS2 cells), and the obtained mixture is further cultured. Thereafter, using a commercially available assay system or kit, luciferase activity is measured.

### (9) Epitope recognized by the antibody of the present invention, and antibody binding to the epitope

The epitope (antigenic determinant) of the antibody of the present invention is not particularly limited, as long as it is at least a portion of the coronavirus S protein as an antigen. As such an epitope, at least a portion of the extracellular region or the receptor-binding domain (RBD) is preferable, and it is, for example, RBD. The antibody that recognizes the aforementioned region (binds to the aforementioned region) has high neutralizing activity against, for example, coronavirus contained in a biological sample, and thus, this antibody is extremely useful for prophylactic or therapeutic use for coronavirus disease.

The epitope of the antibody of the present invention is composed of at least two amino acid residues selected from the group consisting of R403, D405, E406, R408, T415, G416, K417, D420, Y449, Y453, L455, F456, G485, Y486, N487, Y489, Q493, S494, Y495, G496, Q498, N501, G502, V503, G504 and Y505, in the full-length amino acid sequence (for example, SEQ ID NO: 2) of the receptor-binding domain.

Regarding the above-described amino acid residues, the alphabet indicates the single character code of amino acid, and the number indicates the position of the amino acid in the full-length amino acid sequence of the receptor-binding domain. For example, "R403" means the arginine at position 403 in the full-length amino acid sequence (for example, SEQ ID NO: 2) of the receptor-binding domain. The same applies to other positions and amino acid residues, and the amino acid residues can be indicated in this way throughout the present description as a whole.

In one embodiment of the present invention, the antibody of the present invention binds to an epitope comprising the two amino acid residues R403 and K417.

### (10) Binding affinity and neutralizing activity

Binding affinity can be determined by a coupling constant (KA) and a dissociation constant (KD). An affinity equilibrium constant (K) is indicated with the ratio of KA/KD. Such binding affinity can be detected as follows.

The binding affinity has a dissociation constant (KD) of at least 1 × 10⁻¹⁰ M, and the present antibody has an affinity that is, for example, 2 to 5 times, 5 to 10 times, 10 to 100 times, 100 to 1000 times, or 1000 to 10,000 times higher than the aforementioned dissociation constant. Specifically, the antibody of the present invention has a dissociation constant (KD) regarding the binding affinity of the S protein of coronavirus that is preferably 1 × 10⁻¹⁰ M, 5 × 10⁻¹¹ M, 1 × 10⁻¹¹ M, 5 × 10⁻¹² M, 1 × 10⁻¹² M, 5 × 10⁻¹³ M, 1 × 10⁻¹³ M, 5 × 10⁻¹⁴ M, 1 × 10⁻¹⁴ M, 5 × 10⁻¹⁵ M, or 1 × 10⁻¹⁵ M, and is more preferably 1 × 10⁻¹² M to 1 × 10⁻¹⁵ M. Otherwise, the antibody of the present invention may have high affinity that is, however, a value lower than the aforementioned KD values.

Herein, when dissociation constant (KD) of a certain antibody as a measurement subject of the affinity is about 1 to 100 times higher than the KD of the antibody of the present invention, the certain antibody is considered to be substantially identical to the antibody of the present invention, and is included in the present invention.

The coupling constant (KA) and the dissociation constant (KD) can be measured using surface plasmon resonance (SPR), and a known apparatus and a known method, which detect the binding rate in real time and further monitor it, can be adopted (for example, Biacore (registered trademark) T200 (GE Healthcare), ProteON XPR36 (Bio-Rad), etc.).

The neutralizing activity of the antibody of the present invention against coronavirus is less than 2 ng/ml (for example, less than 1ng/ml) as an IC50 value that is an indicator of suppression of viral proliferation.

### 3. Pharmaceutical composition

The pharmaceutical composition of the present invention comprises the antibody of the present invention as an active ingredient, and is useful for the prophylaxis and treatment of SARS virus infection. Further, in order to allow the present pharmaceutical composition to pass through the blood-brain barrier (BBB), a peptidized antibody, or a conjugate of such a peptide and a BBB-permeable carrier may also be produced.

The antibody of the present invention can be administered alone or together with a pharmacologically acceptable carrier, a diluent, and the like. In addition, the antibody of the present invention can be administered once or divided over several administrations.

The pharmaceutical composition of the present invention can be formulated according to a method known to those skilled in the art. For example, a sterile solution of the present pharmaceutical composition with water or a pharmaceutically acceptable solution other than water, or a suspension agent of the present pharmaceutical composition can be parenterally used in the form of an injection. For example, the present pharmaceutical composition is appropriately combined with a pharmacologically acceptable carrier or medium, specifically, with sterilized water or a normal saline, vegetable oil, an emulsifier, a suspending agent, a surfactant, a stabilizer, a flavor, an excipient, a vehicle, an antiseptic, a binder, etc., and is mixed therewith in a unit dosage form required for a generally accepted pharmaceutical practice, so as to obtain a formulation. The amount of an active ingredient in such a formulation is determined, such that an appropriate volume can be obtained in a designated range.

A sterile composition for infection can be formulated using a vehicle such as distilled water for injection according to an ordinary pharmaceutical practice. Examples of an aqueous solution for injection may include a normal saline, and an isotonic solution comprising glucose or other auxiliary drugs (e.g. D-sorbitol, D-mannose, D-mannitol, and sodium chloride). A suitable solubilizer, such as, for example, alcohol (ethanol, etc.), polyalcohol (propylene glycol, polyethylene glycol, etc.), or a nonionic surfactant (polysorbate (TM), HCO-50, etc.) may also be used in combination.

Examples of an oily liquid may include sesame oil and soybean oil. Benzyl benzoate and/or benzyl alcohol may also be used as solubilizers in combination with the oily liquid. In addition, the present pharmaceutical composition may also be mixed with buffers (e.g. a phosphate buffer and a sodium acetate buffer), soothing agents (e.g. procaine hydrochloride), stabilizers (e.g. benzyl alcohol and phenol), and antioxidants. A prepared injection is generally filled into a suitable ampule.

The pharmaceutical composition of the present invention is preferably administered via parenteral administration. For example, an injection type, transnasal administration type, transpulmonary administration type, or transdermal administration type composition can be formulated. Such a pharmaceutical composition can be administered systemically or locally, via intravenous injection, intramuscular injection, intraperitoneal injection, subcutaneous injection, etc.

The administration method can be selected, as appropriate, depending on the age and symptoms of a patient. The applied dose of the pharmaceutical composition containing the antibody or a polynucleotide encoding the antibody can be determined, for example, in the range of 0.0001 mg to 1000 mg per administration per kg of body weight. Otherwise, the applied dose of the pharmaceutical composition can also be determined to be, for example, 0.001 to 100000 mg for a single patient, but the present invention is not necessarily limited to these numerical values. The applied dose and the administration method are changed depending on the body weight, age, symptoms, etc. of the patient. Taking into consideration these conditions, those skilled in the art could determine an appropriate dose and an appropriate administration method of the present pharmaceutical composition.

In the case of oral administration, various excipients such as microcrystalline cellulose, sodium citrate, calcium carbonate, dipotassium phosphate and glycine can be used together with a disintegrator, a binder and the like. Examples of the disintegrator may include starch, alginic acid, and certain types of double silicic acid salts; and examples of the binder may include polyvinylpyrrolidone, sucrose, gelatin, and gum Arabic. Moreover, lubricants such as magnesium stearate, sodium lauryl sulfate, and talc are extremely useful for formation of tablets. When the present pharmaceutical composition is processed into an aqueous suspension or an elixir for use in oral administration, the present pharmaceutical composition is used in combination with an emulsifier and a suspending agent, as necessary, and can be used together with a diluent such as water, ethanol, propylene glycol or glycerin, or a combination thereof.

In the case of a transdermal agent, many ultrafine pores (holes) are opened in the stratum corneum on the surface of the skin, and an adhesive tape, to which a drug has been added, is attached to the skin, so that it has become possible for the skin to absorb the antibody, which has not previously been absorbed through the skin. By adjusting the number and size of holes and the composition of the tape formulation, it has become possible to administer the drug in a short time, like an injection, or to release the drug over a long period of time.

### 4. Reagent and kit for detection or diagnosis of coronavirus

The detection or diagnostic reagent of the present invention contains the antibody of the present invention or a fragment thereof. Since the antibody of the present invention can specifically bind to coronavirus, the reagent of the present invention containing the present antibody can be used for detection or diagnosis of coronavirus disease.

Examples of the coronavirus that becomes a target of the detection or diagnosis in the present invention may include SARS-CoV2, SARS-CoV1, and MERS-CoV.

The reagent of the present invention may comprise a pharmaceutically acceptable carrier, in addition to the antibody of the present invention. The " pharmaceutically acceptable carrier" indicates any given carrier (a liposome, a lipid vesicle, a micelle, etc.), a diluent, an excipient, a wetting agent, a buffer agent, a suspending agent, a lubricant, an adjuvant, an emulsifier, a disintegrator, an absorbent, a preservative, a surfactant, a coloring agent, a flavoring agent, and the like, which are suitable for the reagent of the present invention.

In another embodiment, the present invention provides use of a human antibody against coronavirus or a fragment thereof for the production of an agent for detecting or diagnosing coronavirus disease.

### 5. Method for detecting or diagnosing coronavirus disease

The method for detecting or diagnosing coronavirus disease of the present invention comprises a step of allowing the human antibody of the present invention or a fragment thereof to come into contact with a biological sample collected from a subject (hereinafter, simply referred to as a "biological sample" at times), and detecting a coronavirus protein in the sample. In this case, the detection results of a coronavirus protein are preferably correlated with the possibility of coronavirus disease (be positive to coronavirus infection).

In the present invention, examples of the "subject" may include mammals such as a human, a cat, a dog, a goat, a swine, sheep, a bovine, a horse, a monkey and a hamster, and the subject is preferably a human. When the subject is a human, the subject may include a healthy subject and a patient who has been tested positive to coronavirus infection.

Moreover, the "biological sample" means, for example, mammal-derived tissues and cells, a disintegrated product or an extract of them, a body fluid, an excrement, or a sample containing them, and thus, the biological sample is not particularly limited. Furthermore, the biological sample may also include a sample obtained by performing any given treatment such as, for example, dilution, separation or protein denaturation on mammal-derived tissues and cells, a disintegrated product or an extract of them, a body fluid, and an excrement. The biological sample is preferably a body fluid, and is particularly preferably saliva, mucous membrane, or blood. All of these biological samples can be applied by being subjected to a suitable pre-treatment.

In the present invention, the term "contact" means that the antibody of the present invention and a biological sample collected from a subject are allowed to exist in an identical reaction system. Examples of the contact may include the mixing of the antibody of the present invention with a biological sample in a reaction well, addition of the antibody of the present invention to the biological sample, and addition of either the antibody of the present invention or the biological sample to a carrier on which the other is immobilized.

In the present invention, for the detection of coronavirus, or the measurement or evaluation of the expression level thereof, for example, enzyme immunoassay (EIA, ELISA), fluorescence immunoassay (FIA), radioimmunoassay (RIA), chemiluminescence immunoassay (CIA), a latex agglutination method, a Western blotting method, immunostaining, an immunoprecipitation method, immunochromatography, fluorescence polarization immunoassay (FPIA), etc. can be utilized.

The device used in such detection or the like is not particularly limited, and for example, a microwell plate, an array, a chip, a flow cytometer, a surface plasmon resonance device, an immunochromatography strip, etc. can be utilized. When such a device is used, signals of a coloring level, a fluorescence level, a luminescence level, etc. are detected, measured and/or evaluated, and the obtained detection, measurement or evaluation results can be collated with the possibility of coronavirus disease, etc.

In the present invention, based on the detection results of coronavirus (or the measurement results of the expression level thereof), the presence or absence of the possibility that a subject is affected with coronavirus disease (i.e. the presence or absence of being tested positive) can be diagnosed.

In this case, the antibody of the present invention is labeled with an enzyme or a fluorescent substance, so that the reaction may be directly detected as a fluorescence signal. Otherwise, such a signal may be indirectly detected by using a labeled secondary antibody that binds to the antibody of the present invention. Alternatively, as such a detection method, any method such as a competitive method, a sandwich method or a direct adsorption method may be applied. Then, the reaction intensity, or the percentage of viruses binding to the antibody in all viruses contained in the reacted sample is measured, then, the previously set reference value or indicator is determined to be a cut-off value, and the actually measured value is then compared with the cut-off value, so that the possibility that the subject is affected with coronavirus disease can be judged or diagnosed.

The cut-off value can be obtained, for example, as follows. First, the expression level of coronavirus in a biological sample derive from a coronavirus-positive patient, and the expression level of coronavirus in a biological sample derived from a healthy subject, are measured (in this case, the obtained value is predicted to be below the detection limit). In this measurement, the number of patients who become subjects is 2 or more in both cases, and it is, for example, 10 or more, or 100 or more. Then, from both the positive patient-derived biological sample group and the healthy subject-derived biological sample group, the cut-off value of the expression level of coronavirus is obtained by a statistical treatment. As such a statistical treatment, for example, a logistic regression analysis method, a method of using a ROC curve, or the like can be used.

In the present invention, the critical value (cut-off value) of the expression level of a coronavirus S protein (extracellular region) in a blood sample can be determined, as appropriate, as a concentration in serum.

In a case where the expression level of a coronavirus S protein is measured under the above-described conditions, when the expression level is the above-described cut-off value or greater, it can be determined that the coronavirus has been detected, or it can be diagnosed that the subject is likely to be positive to the coronavirus.

In the present invention, the certainty (probability) of the detection results obtained by detecting a coronavirus S protein is 60% or more, 70% or more, 80% or more, 90% or more, 95% or more, and preferably 99% or more.

The above detection results can be used as main or supplementary materials, when the examination of coronavirus disease or the definitive diagnosis of therapeutic effects is carried out.

Specifically, the present invention also provides a method for assisting detection or diagnosis of coronavirus disease, comprising a step of allowing a human antibody against a coronavirus S protein or a fragment thereof to come into contact with a biological sample collected from a subject, and detecting a coronavirus S protein in the sample.

Hereafter, the present invention will be described in detail in the examples below. However, the present invention is not limited to these examples.

### Examples

Hereinafter, the present invention will be more specifically described in the following examples. However, these examples are not intended to limit the scope of the present invention.

### [Example 1]

### 1) Production of recombinant antigens

The nucleotide sequences of the extracellular region and RBD region proteins of recombinant S proteins of SARS-CoV2, SRAR-CoV1 and MERS-CoV were artificially synthesized, and the synthesized nucleotide sequences were each inserted into a mammalian expression vector (pCMV vector), into which a protein purification tag (9 x His tag) had been inserted, thereby producing individual recombinant protein expression vectors. The thus produced expression vectors were each introduced into Expi293 cells (Thermo Fisher Scientific), and the resulting cells were then cultured at 37°C in 8% CO₂ by a shaking culture at 125 rpm for 5 days.

Each recombinant protein was purified from the culture supernatant, using TALON Column (Clontech).

### 2) Immunization of complete human antibody-producing mice (TC mice) with recombinant virus antigen

10 µg of a SARS-CoV2 recombinant S protein (an extracellular region, an RBD region) was mixed with 100 µl of a Freund's incomplete adjuvant and 10 µg of phosphorothioated CpG (ATAATCGACGTTCAAGCAAG (SEQ ID NO: 21)). The mixed solution was injected into the abdominal cavity of complete human antibody-producing mice (TC mice). A SARS-CoV1 recombinant S protein (an extracellular region, an RBD region) and a MERS recombinant S protein (an extracellular region, an RBD region) were prepared by the same method as that described above, and booster was carried out every two weeks.

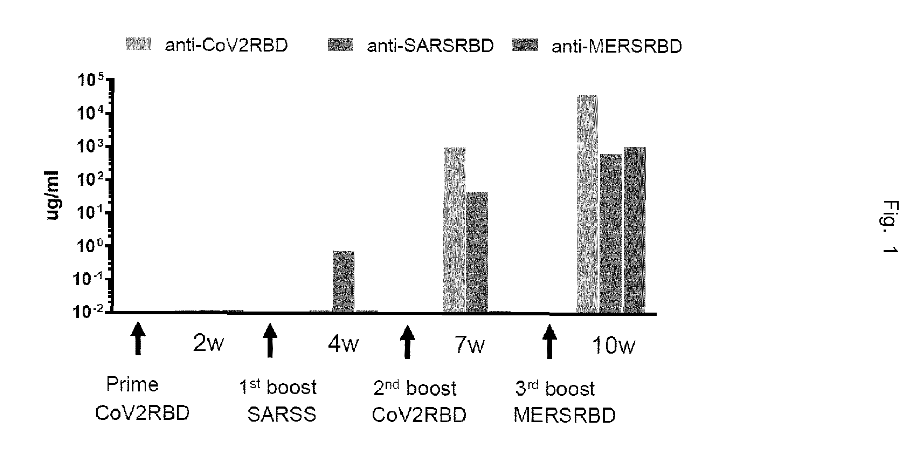

The immunization schedule is shown in Figure 1.

### 3) Production of RBD antigen probes

Fluorescent dyes (PE (DOJINDO CO., LTD.), APC (DOJINDO CO., LTD.), AlexaFluor594 (Thermo Fisher Scientific), and PerCP-Cy5.5 (abcam)) were allowed to bind to the recombinant S proteins (an extracellular region and an RBD region) of SARS-CoV2, SARS-CoV1 and MERS-CoV, so as to produce fluorescent probes.

### 4) Fractionation of S protein-binding memory B cells from TC mice and isolation of monoclonal antibody

Blood was collected from TC mice, and the antibody titers of blood IgGs against the S proteins (an extracellular region, an RBD region) of SARS-CoV2, SARS-CoV1 and MERS-CoV were then measured according to sandwich ELISA. Splenic cells were collected from mice, in which the IgG antibodies were induced to all of the S proteins.

The splenic cells were treated with an anti-FcyRII/III antibody, and thereafter, the antibodies described below, and memory B cells that were stained with S protein fluorescent probes and bound to the S protein fluorescent probes, were fractionated by flow cytometry.

The fractionated memory B cells were dispersed in an amount of 1 cell/well on feeder cells in which mouse CD40L and BAFF were expressed, and the obtained mixture was then cultured using an RPMI medium supplemented with mouse IL-2 (4 ng/mL), IL-4 (2 ng/mL) and IL-5 (5 ng/mL) under conditions of 37°C and 5% CO₂. On Day 10 after the culture, a culture supernatant was recovered.

### 5) Production of antibody proteins

cDNA was synthesized from memory B cells which had been confirmed to have binding ability to individual S proteins according to an ELISA method, and antibody genes (heavy chain and light chain V regions) were then cloned according to a PCR method.

The cDNA was synthesized as follows.

RNA prepared from the cells using RNeasy Plus Mini Kit (QIAGEN) was subjected to cDNA synthesis (42°C, 120 minutes) using SuperScript^{™} VILO^{™} Master Mix (Invitrogen).

The PCR was carried out using the following reagent and cycle conditions.

PrimeSTAR (registered trademark) Max DNA Polymerase (TAKARA) was used, and amplification was carried out under conditions of 98°C (5 seconds), 55°C (5 seconds) and 72°C (30 seconds), and 30 cycles.

The cloned heavy chain V region was introduced into a mammalian expression vector (pIg vector) comprising human IgG1, whereas the cloned light chain V region was introduced into a mammalian expression vector (pIg vector) comprising human Igkappa or Iglambda C region, thereby producing individual antibody expression vectors.

The thus produced antibody expression vectors were each introduced into Expi293 cells (Thermo Fisher Scientific), and recombinant antibodies were then purified from culture supernatants, using Protein G.

### 6) Confirmation of binding ability of antibodies according to ELISA

According to an ELISA method, the binding of monoclonal antibodies to SARS-CoV2 RBD, SARS-CoV1 RBD and MARS-CoV RBD was measured. Hereinafter, a detailed example of the ELISA method will be described.

SARS-CoV2 RBD, SARS-CoV1 RBD or MARS-CoV RBD was dissolved in a phosphate-buffered saline (pH 7.3), and the obtained solution was then added in an amount of 50 µL each onto a 96-well plate. The 96-well plate was left at rest at 4°C overnight, and individual wells were then washed with a phosphate-buffered saline three times. After that, 110 µl each of a phosphate-buffered saline containing 1% bovine serum albumin was added to individual wells. The plate was left at rest at room temperature for 1.5 hours, and individual wells were then washed with a phosphate-buffered saline (containing 0.1% Tween 20) three times. Thereafter, a monoclonal antibody that had been serially diluted with a phosphate buffer containing 0.1% Tween 20 and 1% bovine serum albumin was added in an amount of 50 µL each onto the plate. The plate was left at rest at 4°C overnight, and individual wells were then washed with a phosphate-buffered saline (containing 0.1% Tween 20) three times. After that, a peroxidase-labeled anti-human IgG antibody (Southern Biotech), which had been serially diluted with a phosphate-buffered saline containing 0.1%Tween 20 and 1% bovine serum albumin, was added in an amount of 50 µL each into individual wells. The plate was left at rest at room temperature for 1 hour, and individual wells were then washed with a phosphate-buffered saline (containing 0.1% Tween 20) three times. Thereafter, 20 mL of a citrate buffer (pH 5.0), to which 10 mg of o-phenylendiamine tablets (SIGMA-ALDRICH) and 8 µL of a 30% hydrogen peroxide solution (Wako Pure Chemical Industries, Ltd.) were added, was prepared as a substrate, and the prepared solution was then added in an amount of 100 µL each into individual wells. After color development, the reaction was terminated with 50 µl of 2 N sulfuric acid (Wako Pure Chemical Industries, Ltd.), and the absorbance value at 490 nm was then measured using Microplate Reader 450 type (Bio-Rad).

### 7) Neutralization test

In order to evaluate the SARS-CoV2 virus infection-protecting effect of a monoclonal antibody, a neutralization test was carried out using VSV pseudovirus. Hereafter, a detailed example of the neutralization test will be described.

A monoclonal antibody, which had been serially diluted with a DMEM medium (containing 10% fetal bovine serum and 0.1% penicillin/streptomycin), was mixed with a CoV2 S protein-expressing VSV pseudovirus, which had been diluted with a DMEM medium, and the obtained mixture was then reacted in an incubator (5% CO₂, 37°C) for 1 hour. After completion of the culture, the mixed solution of the antibody and the virus was added to Vero E6/TMPRSS2 cells cultured in a 96-well plate. The thus obtained mixture was cultured in an incubator (5% CO₂, 37°C) for 24 hours, and 10 µL of Bright-Glo Luciferase Assay System (Promega) was then added to individual wells. Thereafter, luciferase activity was measured using GloMax NAVIGATOR (Promega).

The results are shown in Figures 2 to 4.

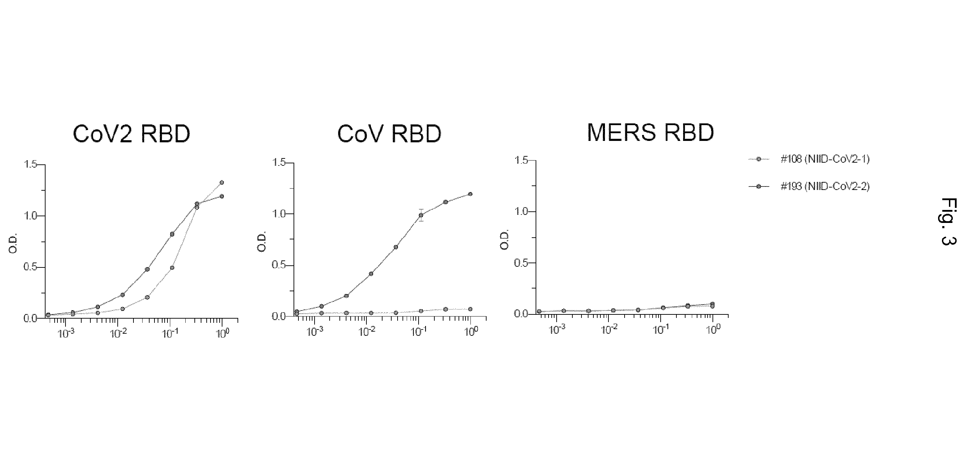

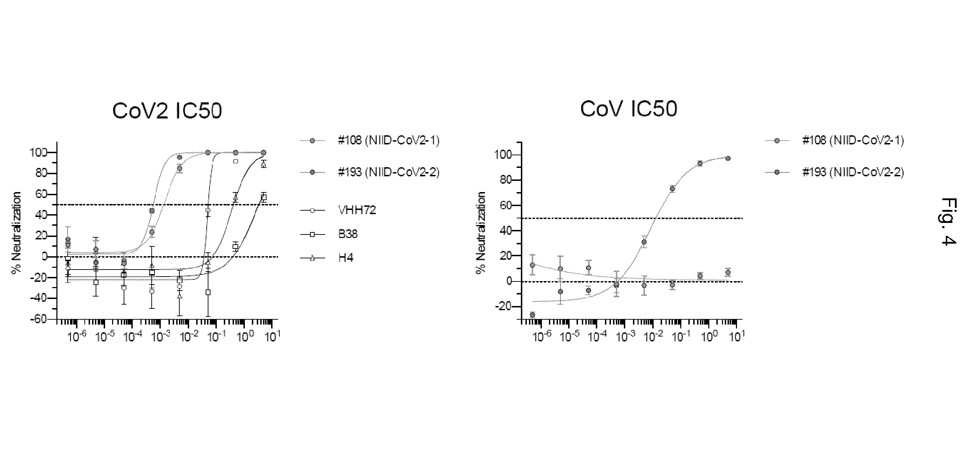

Figure 2 is a view showing the results of separation of memory B cells. Figure 3 is a view showing the results of isolation of antibodies against individual coronaviruses. Figure 4 is a view showing the results obtained by measuring the neutralizing activity of antibodies, using pseudo viruses.

In Figure 3, #108 (NTCOV2-1) is specific to SARS-CoV2, and #193 (NTCOV2-2) exhibits cross-reactivity to SARS-CoV2/CoV1.

In Figure 4, with regard to the IC50 of the neutralizing activity of each antibody against SARS-CoV2, NTCOV2-1 had an IC50 value of 1.3 ng/mL, NTCOV2-2 had an IC50 value of 0.56 ng/mL, VHH72 had an IC50 value of 52 ng/mL, B38 had an IC50 value of 3460 ng/mL, and H4 had an IC50 value of 426 ng/mL.

In addition, with regard to the IC50 of the neutralizing activity of each antibody against SARS-CoV, NTCOV2-2 had an IC50 value of 13 ng/mL.

### [Example 2]

### Bimodal strategy of SARS-CoV-2 antibody for increasing neutralization width and escape resistance

### (1) Outline

The receptor-binding site (RBS) of SARS-CoV-2 is an important target of a strong neutralizing antibody. Epitope variability hinders cross-neutralization of multiple coronaviruses and resistance to escape mutants. In the present study, there was identified a human RBS antibody that super strongly cross-neutralizes both SARS-CoV-2 virus and SARS-CoV virus. Cross-neutralization was achieved by bimodal binding, namely, the binding of a heavy chain to a preservation site and the binding of a light chain to RBS. A unique binding mode enabled a wide range of RBS masking through a minimum interaction with a hypervariable region, and also enhanced resistance to antibody escape mutants including novel interesting mutants. Further, an antibody treatment provided protection to hamster models under prophylactic and therapeutic conditions. In the present example, there is elucidated a novel antibody strategy for acquiring wide and strong neutralizing activity that means a therapeutic drug and a vaccine for achieving a wide range of neutralization.

The SARS-CoV-2 (CoV2) pandemic has caused one hundred million or more of infections and 2 million or more of dead people over the world, as of January 27, 2021 (www.who.int). From individual mice in the convalescence stage and humanized mice, more than 400 CoV2 neutralizing antibodies were isolated (Reference 1), and several epitope structures were resolved to induce effective vaccines and therapeutic drugs (Reference 2). A representative target of such a super strong neutralizing antibody is the receptor-binding site (RBS) of a spike (S) protein that is directly contacted with angiotensin-converting enzyme 2 (ACE2) in human cells (Reference 3). If the preservability of RBS sequences is low between CoV2 and other coronaviruses (CoVs) including SARS-CoV (CoV1), the possibility of cross-neutralization of the RBS antibody against multiple CoVs decreases. Further, the existing RBS antibodies are sensitive to escape mutation under the use of monotherapy (References 4 to 6). Accordingly, since such structural data promote a novel antibody therapy and vaccine designing, a CoV2 RBS antibody having neutralization width and escape resistance is required.

### (2) Materials and methods

### Recombinant S protein antigens

A human codon-optimized nucleotide sequence (GenBank: MN994467; nucleotides: 21563 to 25384 (SEQ ID NO: 22)) encoding the spike (S) protein of a SARS-CoV-2 isolate was commercially synthesized (Eurofinsgenomics). RBD (amino acids: 322 to 536 (SEQ ID NO: 23)), together with a signal peptide (amino acids: 1 to 20; MIHSVFLLMFLLTPTESYVD (SEQ ID NO: 24)) and a histidine tag, was cloned into the mammalian expression vector pCAGGS. A soluble version (amino acids: 1-1201 (SEQ ID NO: 34)) of a spike protein comprising a T4 folden trimerization domain, a histidine tag, and a strep-tag was cloned into the mammalian expression vector pCMV. The amino acid sequence of the protein was modified to remove a multibasic cleavage site ( i.e. remove A from RRAR), and two stabilization mutations were also introduced (K986P and V987P; wild type numbering) (Reference 32).

Also, the spike proteins of SARS-CoV-1 (GenBank: EU371563; amino acids: 15-1195 (SEQ ID NO: 25)), MERS-CoV (GenBank: KF192507; amino acids: 13-1296 (SEQ ID NO: 26)), HCoV-NL63 (GenBank: NC_005831; amino acids: 22-1293 (SEQ ID NO: 27)), HCoV-OC43 (GenBank: NC_006213; amino acids: 12-1299 (SEQ ID NO: 28)), HCoV-229E (GenBank: NC_002645; amino acids: 12-1109 (SEQ ID NO: 29)), and HCoV-HKU1 (GenBank: NC_006577; amino acids: 13-1293 (SEQ ID NO: 30)) were each cloned into a pMT vector comprising a T4 folden trimerization domain, a histidine tag, and a strep-tag.

Also, the RBDs of the S proteins of SARS-CoV-1 (amino acids: 310-509) and MERS-CoV (amino acids: 367-606), together with a histidine tag, were each cloned into the mammalian expression vector pHLsec. RBD mutants of SARS-CoV-2 involving the positions A475V, E484K, Q493R, R346S, N440K, and Y453F were produced according to site-directed mutagenesis using primers, into which Gibson assembly (NEB) had been introduced based on related nucleotide substitutions and RBD expression vectors. Recombinant proteins were produced using a fruit fry expression system or Expi293F cells (Thermo Fisher Scientific). On Day 5 after the transfection, a supernatant was collected from the transfected cells, and recombinant proteins were then purified using Ni-NTA agarose (Qiagen) and/or streptactin sepharose (Sigma Aldrich). In order to produce S protein-fluorescent dye probes, the recombinant RBD and S protein of SARS-CoV-2 were allowed to bind to APC and PE (Dojindo).

### Mice and immunization

TC-mAb mice were established as described above (Reference 7). Mice were immunized via i.p. with the recombinant RBD (10 µg/body) of a SARS-CoV-2 spike protein, together with the adjuvant AddaVax (InvivoGen). Thereafter, the mice were continuously boosted with the recombinant RBDs or S proteins of SARS-CoV-1, SARS-CoV-2 and MERS-CoV with intervals of 21 days. On Day 7 after the booster, serum was collected, and the antibody titer of the antigen-specific human IgG antibody was then measured. All of the mice were maintained under SPF conditions, and were used when the mice were 7 to 14 weeks old. All operations were performed on the mice in accordance with the guidelines of the Animal Care and Use Committee, Japan National Institute of Infectious Diseases.

### ELISA method

An ELISA plate was coated with any of the RBDs of the S proteins of SARS-CoV-1, SARS-CoV-2 and MERS-CoV, the RBD of SARS-CoV-2, and the RBD mutants of SARS-CoV-2, E484K, Q493R, R346S, N440K and Y453F, or the trimeric S proteins of SARS-CoV-1, SARS-CoV-2, MERS-CoV, HCoV-NL63, HCoV-OC43, HCoV-229E and HCoV-HKU1, in a concentration of 2 µg/ml each. In several experiments, the concentrations of the coating antigens were changed to 0.2 µg/ml or 5 µg/ml. After blocking with PBS containing 1% BSA, serially diluted serum, a culture supernatant of single cells, or mAb was applied onto the plate, and the resultant was then incubated together with goat anti-human IgG-HRP (Southern Biotech, Cat#: 2040-05, 1 : 5000). HRP activity was visualized with OPD substrate (Sigma) 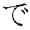 , and OD490 was then measured using iMark Microplate Reader (Bio-Rad).

### Flow cytometric analysis

The splenic cells of TC-mAb mice were pre-treated with anti-FcyRIVIII mAb, and were then incubated with biotinylated mAb against mouse CD43, CD90, CD3, c-kit, F4/80, Gr-1, CD4, CD8, CD11b, Ter119, CD93, CD11c, CD138 and human IgD. Class-switched memory B cells were concentrated by MACS system using streptavidin microbeads (Miltenyi Biotec). Following this operation, the cells were stained with B220-BV786, CD38-AF700, PE-labeled CoV2-S, APC-labeled CoV2-RBD, streptavidin-efluor450, and DAPI. The stained cells were analyzed, and were then sorted with single cells on a 96-well plate, using FACS Aria device (BD Bioscience).

### Single cell culture

One day before the sorting of the single B cells, feeder cells (NB21.2D9 cells) in a B cell medium (BCM; RPMI-1640 supplemented with 10% Hyclone FBS, 1 mM sodium pyruvate, 10 mM HEPES, MEM non-essential amino acids, 100 IU/mL penicillin, 100 µg/mL streptomycin, and 55 µM 2-mercaptoethanol) were seeded on a 96-well plate, in an amount of 1000 NB21.2D9 cells per well. On the following day, mouse IL-4 (Peprotech: 2ng/mL) in BCM was added to the culture, and mouse/human B cells were directly sorted out on the 96-well plate in an amount of 1 cell/well, followed by performing a co-culture with the feeder cells. After completion of the culture, the culture supernatant was recovered, and was then subjected to ELISA for the mapping of epitopes of the secreted mAbs. Moreover, the cultured clone B cells were frozen, and V(D)J sequence analysis for production of recombinant mAbs and the recovery of V(D)J genes were carried out.

### Generation of mAbs

A recombinant monoclonal antibody was prepared based on the findings of the previous reports (References 8 and 33). Briefly, the single cell cultures or the V_{H}/V_{L} genes of the B cell sample selected from the published mAbs (REGN10933, REGN10987, and S309) were cloned into human IgG1, IgG2, IgG3, or IgG4 heavy chain and κ or λ light chain expression vectors. A pair of the heavy chain and light chain vectors was transfected into Expi293F cells (Thermo Fisher Scientific; Cat#: A14527) in accordance with the manufacturer's instructions. Subsequently, using Protein G Column (Thermo Fisher Scientific), an antibody was purified from the culture supernatant, and was then dialyzed against PBS, and the resultant antibody was subjected to further analyses.

### Inhibition of binding to ACE2

In order to determine inhibition of the binding of SARS-CoV2 RBD to ACE2 by an antibody, recombinant human ACE2 (Biolegend) was coated on a 96-well plate (Corning) at 4°C overnight. After blocking with PBS containing 1% BSA, recombinant SARS-CoV2 RBD having Avitag was pre-incubated together with a serially diluted antibody at RT for 2 hours. Thereafter, the obtained mixture was transferred into wells of the ACE2-coated plate, and was then incubated at 4°C overnight. The binding of SARS-CoV2 RBD to ACE2 was developed using streptavidin-HRP (Southern Biotech) and OPD-substrate (Sigma-Aldrich). The absorbance at 490 nm was read using a microplate reader (BioRad). The inhibition percentage was calculated according to the formula (O.D. value of positive control - O.D. value of sample) / (O.D. value of positive control - O.D. value of negative control) x 100. The half maximal effective concentration (EC50) was determined using nonlinear regression curve fitting (GraphPad Prism).

### Virus neutralization assay

For a pseudovirus neutralization assay, VSV pseudovirus having a SARS-CoV2 or SARS-CoV1 spike protein was incubated together with a serially diluted antibody at 37°C for 1 hour. Thereafter, Vero E6 or Vero E6/TMPRSS2 cells seeded on a 96-well solid white flat-bottom plate (Corning) were inoculated onto the reaction mixture, and the thus obtained mixture was then incubated with 5% CO2 at 37°C for 24 hours. Employing GroMax Navigator Microplate Liminomater (Promega), luciferase activity in the cultured cells was measured using Bright-Glo^{™} Luciferase Assay System (Promega). The neutralization percentage was calculated according to the formula (virus control signal - sample signal) / (virus control signal - control signal of only cells) x 100. The half maximal inhibition concentration (IC₅₀) was determined using nonlinear regression curve fitting (GraphPad Prism).

For an authentic virus neutralization test, a 100 TCID₅₀ SARS-CoV-2 JPN/TY/WK-521 stain and a serially diluted antibody were incubated at 37°C for 1 hour, and the obtained mixture was then placed on Vero E6/TMPRSS2 cells (JCRB1819; JCRB Cell Bank) that had been seeded on a 96-well flat bottom plate (TPP). After completion of the culture at 37°C in 5% CO₂ for 5 days, the resultant was immobilized with 20% formalin (Fuji Film) and was then stained with a crystal violet solution (Sigma-Aldrich). Thereafter, the absorbance at 595 nm was measured using Epoch 2 (Biotek). The neutralization percentage was calculated according to the formula (sample signal - virus control signal) / (control signal of only cells - virus control signal) x 100.

### SPR

The full-length fragments and Fab fragments of NT-108 and NT-193 were each dissolved in a HBS-EP buffer (10 mM HEPES pH 7.4, 150 mM NaCl, 3 mM EDTA, and 0.005% surfactant P20). An SPR experiment was carried out using Biacore 3000 or T200 (Cytiva). A biotinylated S-RBD protein was poured onto a streptavidin-immobilized CM5 sensor chip or CAP chip (Cytiva) according to amine coupling using a standard amine coupling kit, so that the biotinylated S-RBD protein was immobilized thereon. As a negative control protein, β2-microglobulin was used. An NTCOV antibody was poured at a flow rate of 30 µl/min onto the immobilized S-RBD protein. The data were analyzed using BIA evaluation version 4.1.1, T200 evaluation version 1.0, and ORIGIN version 2017 software.

### Crystallization, data collection, and structure determination

Crystals of an S-RBD - NT-108 complex were allowed to grow at 293K according to a sitting drop vapor diffusion method. The final crystallization conditions were 0.1 M HEPES pH 7.5, 10% (w/v) PEG 8000, and 8% (w/v) ethylene glycol. The crystals were frozen in liquid nitrogen, and an X-ray diffraction experiment was carried out with the beam line BL-17A in a photon factory (Tsukuba, Japan). An X-ray diffraction data set was treated with XDS, and was then scaled with Aimless in a CCP4 program package. The structure was elucidated according to a molecular replacement method of using a Phaser program of a PHENIX package, in which the S-RBD structure (PDB ID: 7 jmp) was used as a search probe, and thereafter, structure refinement was carried out using phenix.refine and COOT. Using Molprobity, the stereochemical properties of the structure were evaluated. In Figure 5-2, Figure 6-7 and Figure 6-8, the complexes were prepared using PyMOL (http://PyMOL.sourceforge.net). Intermolecular contact atoms were analyzed in a CCP4 program package, using PISA and CONTACT.

### In vivo load test

In order to determine the protection of hamster models from SARS-CoV2 virus infection by an antibody treatment, ketamine hydrochloride/xylazine was intraperitoneally injected into Syrian hamsters (i), and were then infected with 80 µL of the SARS-CoV2 virus JPN/TY/WK-521/2020 at a dose of 10⁴ TCID₅₀ via i.n. (intranasal) anesthesia. The antibody was diluted with 5 or 1.25 mg/kg body weight of PBS in a predetermined volume (200 µL), and was then administered to the hamsters via intraperitoneal injection, either 2 weeks before virus challenge for a prophylactic treatment, or 1 day after virus challenge for a therapeutic treatment. All of the hamsters were monitored every day until Day 6 after the infection, in terms of viability and reduction in body weight. Human end point was set to be 25% body weight reduction with respect to the initial body weight upon the virus challenge (Day 0).

### qRT-PCR analysis of viral RNA

The amount of the virus was measured by qRT-PCR for detecting the nucleocapsid genomic RNA of SARS-CoV-2. Using Direct-zol miniprep kit (Zymo580 research, Cat. No. R2050), lung total RNA was extracted from a lung homogenate. Using TissueLyser LT (Qiagen), a whole lung homogenate was prepared. In the qRT-PCR reaction, using QuantiTect Probe RT-PCR kit (Qiagen #204443), the following cycle protocols were applied: 30 minutes, 50°C; 15 minutes, 95°C; then, 15 seconds, 95°C; and 1 minute, 60°C. Replication was carried out by the 45 cycles. The sequences of the primers and probe used in the qRT-PCR are as follows: forward direction: 5'-AAATTTTGGGGACCAGGAAC-3' (SEQ ID NO: 31), reverse direction: 5'-TGGCAGCTGTGTAGGTCAAC-3' (SEQ ID NO: 32), and probe: 5'FAM-ATGTCGCGCATTGGCATGGA-BHQ-3' (SEQ ID NO: 33). A calibration curve is constructed using a serial 10-fold dilution of a purified PCR product having a known copy number (10 to 10⁶ copies/pL).

### (3) Results and consideration, etc.

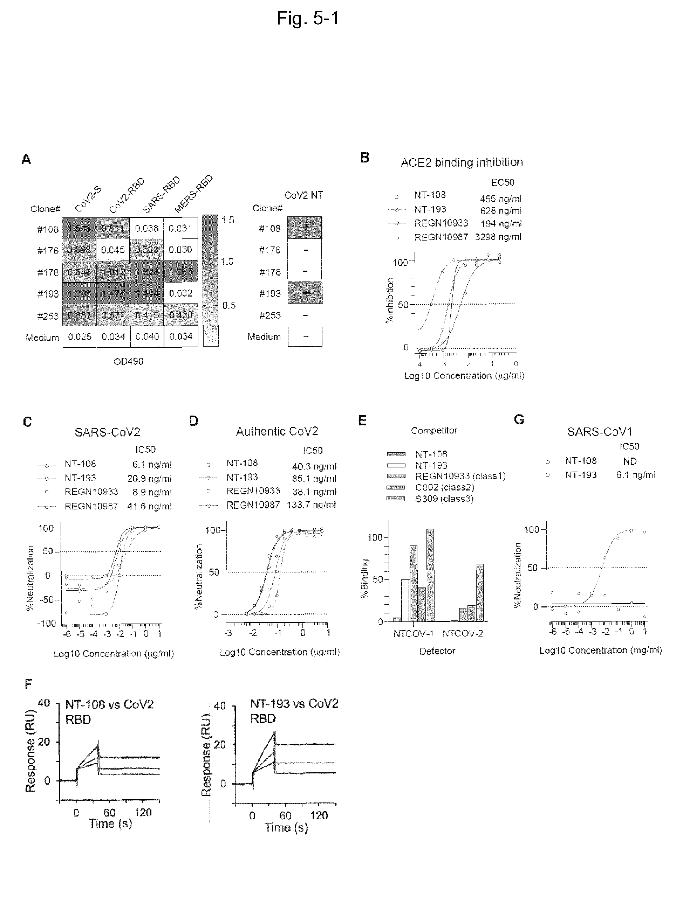

In the present example, there were utilized TC-mAb mice, which stably maintain genetically engineered chromosomes including all human Ig heavy chain and κ chain gene loci under mouse Ig knock-out background (Reference 7). As shown in Figure 6-1, the mice were repeatedly immunized with multiple antigens, and thereafter, B cells binding to the CoV2 receptor-binding domain (RBD) were sorted out, as described above, for use in single cell culture (Reference 8). Among high affinity CoV2 RBD binders, CoV2 neutralizing activity was detected from two clones based on a VSV-based pseudovirus assay (Figure 5-1A). One clone (#108) was specific to CoV2 RBD, and the other clone (#193) was cross-reactive to CoV2/CoV1 RBD. Hereinafter, the #108 and #193 clones are referred to as "NT-108" and "NT-193," respectively.

The RBS antibody is often encoded by public V_{H} genes (Reference 9; Reference 10), whereas NT-108 and NT-193 utilize non-specific IGHV6-1 and IGHV4-34 genes, respectively (Figure 6-2). Among 658 CoV2 RBD antibodies from COVID-19 patients (as of January 17, 2021), only two IGHV6-1-encoded antibodies have been deposited in the CoV-AbDab database (Reference 1). In contrast, a representative V_{H} gene of an autoreactive antibody (Reference 11), IGHV4-34, has been frequently recovered from the B cells of COVID-19 patients (Reference 12), and has been utilized by 11 CoV2 RBD antibodies in the CoV-AbDab database (Reference 1). It is noteworthy that CoV1 cross-reactivity was detected in two of the 11 RBD antibodies encoded by V4-34 (Reference 13), and that this V_{H} gene is likely to promote the CoV1 cross-reactivity.

RBS epitopes can be subdivided by two types of classifications; namely, classes 1 and 2 in one classification (Reference 14), and A, B and C in the other classification (Reference 2). Further, in the spike RBD, a non-RBS epitope shown as either class 3/4 or an S309-latent/CR3022-proteoglycan site is present. In general, a class 4 (CR3022- proteoglycan) epitope is insufficiently targeted or is targeted by non-neutralization clones (Reference 15), and thereby, the present inventors have excluded antibodies against this class from further analyses. A recombinant human IgG1/κ antibody was allowed to express based on NT-108 and NT-193 V_{H} /V_{L} sequences. For comparison, a humanized mouse-derived REGN10933 antibody and a human-derived REGN10987 antibody were also prepared (Reference 4). This is because an antibody cocktail is useful for therapeutic use in animal models and humans (References 16 and 17). Furthermore, an S309 antibody against a class 3 epitope was allowed to express as a representative CoV1/CoV2 cross-neutralizing antibody (References 15 and 18). IgG1 of both NT-108 and NT-193 inhibited the binding of RBD to human ACE2 (Figure 5-1B), but S309 as a non-RBS antibody did not inhibit the binding of RBD to human ACE2 (Figure 6-3). In order to obtain a further insight regarding those epitope classes, competitive ELISA was carried out, in which RBD classes 1-3 epitopes were masked by representative antibodies of individual classes (Figure 5-1C). Strong competition by a class 2 antibody (C002) reinforced that both NT-108 and NT-193 are actually RBS antibodies. A further competition by a class 1 antibody suggests that NT-193 recognizes epitopes including classes 1 and 2. NT-108 and NT-193 probably recognize partially overlapped epitopes when they compete with each other. Importantly, the two antibodies exhibit extremely strong neutralizing activity against CoV2 pseudovirus and authentic virus, and the IC₅₀ was strong at the same level as an REG antibody (1 digit or 2 digits ng/mL) (Figures 5-1D and 5-1E). As demonstrated by a surface plasmon resonance (SPR) analysis, such strong neutralizing activity is supported by significantly high affinity to RBD (apparent affinity: ~ 10^{-11 to-12} M) (Figure 5-1F). Accordingly, both NT-108 and NT-193 are highly neutralizing RBS antibodies, although they do not involve the conventional use of V_{H}.

A clear characteristic between NT-108 and NT-193 is cross-reactivity to CoV1 (Figure 5-1A). The present inventors have further examined the cross-reactivity of these antibodies to MERS and various CoV S trimers including four seasonal CoVs (Figure 6-4). No cross-reactivity to any CoVs was detected from NT-108, and the cross-reactivity of NT-193 was limited to only CoV1. It should be noted that NT-193 bound to CoV1 RBD with high affinity (Figure 6-5), and neutralized CoV1 with intensity equivalent to that for CoV2 virus (Figure 5-1G). CoV1/CoV2 cross-neutralizing antibodies were isolated from several groups (References 13 and 18 to 20). The neutralizing activity of these cross-neutralizing antibodies against either CoV1 or CoV2, or against both of them, is generally weaker than the neutralizing activity of NT-193 (6.1 ng/mL IC₅₀) (>100 ng/mL IC₅₀). Therefore, NT-193 is a unique RBS antibody that neutralizes both the CoV2 and CoV1 viruses, strongly and equivalently.

Only 24±9 S trimers are presented on the surfaces of individual CoV2 virions (Reference 21). As previously observed in HIV-1 (Reference 22), such low-density S proteins are likely to hinder the bivalent binding by antibodies necessary for inter-S protein cross-linking. In the case of HIV-1, an increase in the crosslinking was achieved by an Env-binding antibody associated with an IgG3 subclass, having a long and flexible hinge region (Reference 23). In order to imitate a low-density RBS epitope on a CoV2 virion, an RBD protein was coated at a non-saturated concentration onto an ELISA plate (Figure 6-6). Thereafter, NT-108 and NT-193 of IgG1, IgG2, IgG3 and IgG4 subclasses were added to the plate to evaluate binding activity. A subclass-dependent difference was not observed in a binding curve to the saturated concentration of RBD, but when compared with other IgG subclasses, the amount of the IgG3 subclass that bound to the non-saturated concentration of RBD increased. According to a virus neutralization assay using pseudo and authentic CoV2 viruses, the IgG3 subclass was confirmed to have excellent activity (Figure 6-6).

In order to elucidate the structural aspects of the interaction between the S protein and antibodies, X-ray crystallography was carried out. The present inventors have succeeded in obtaining crystals of a complex of an S-RBD protein and a Fab fragment of NT-193. The structure of the complex was determined by 2.8Å resolution (Table 2-1).

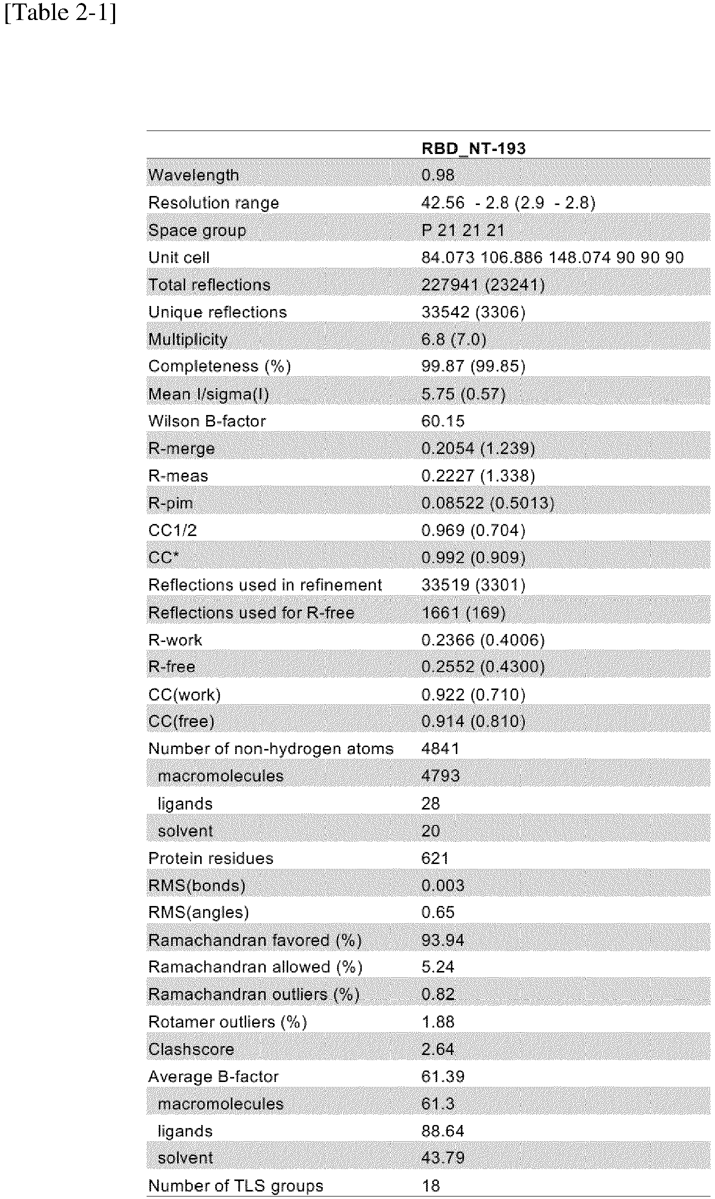

NT-193 recognizes the upper region of S-RBD that is widely overlapped with the binding site of ACE2 (Figure 5-2A). The class 1/RBS-A/B antibody (References 2 and 14) also recognizes the aforementioned upper region, but the recognizing mode of NT-193 is apparently different from that of the class 1/RBS-A/B antibody and is unique (Figure 5-2B and Figure 6-7A). In particular, for recognition of the ACE2 binding site, the NT-193 antibody does not only utilize the CDR-L1 and CDR-L3 of the light chain, but also utilizes the frame region and the DE loop (Figures 5-2C and 5-2D, and Table 2-2).

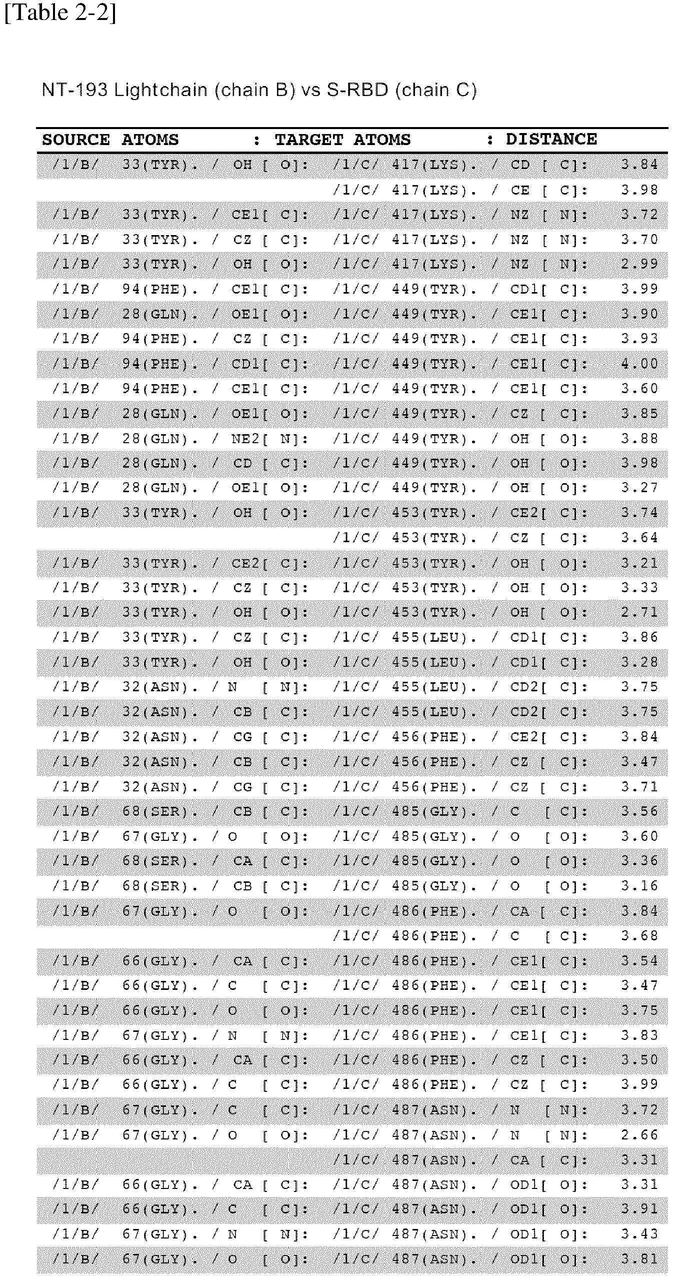

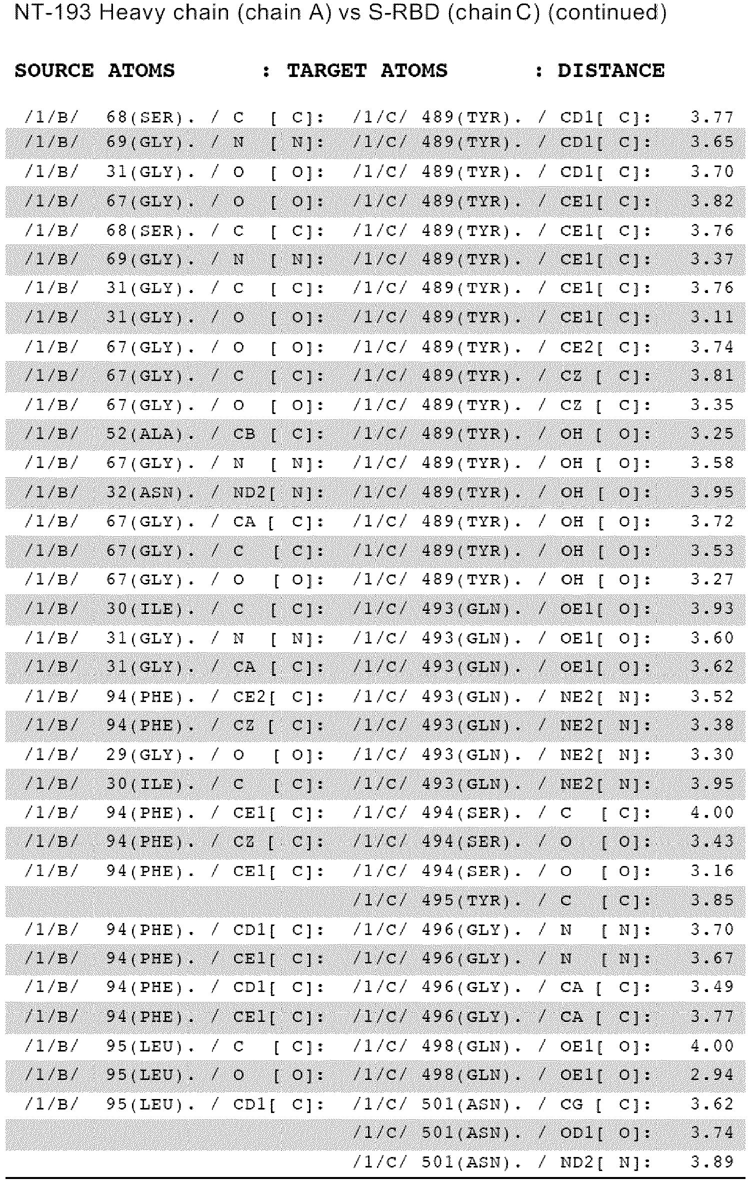

On the other hand, the protruding CDR-H3CDR-H3 of the heavy chain, together with only small parts of CDR-H1 and CDR-H2, binds to a CoV1/CoV2 preserved region adjacent to the ACE2 binding site (Figures 5-2E and 5-2F, and Figures 6-8 and 6-9). These structures are characterized by the non-specific binding mode of an antibody complex (Figure 6-7B), and probably, these characteristics would contribute to strong neutralizing activity and cross-reactivity to CoV2 and CoV1.

Specifically observing the binding interfaces of the CDR regions (Table 2-3), regarding CDR-L3, Phe-94 performs π-π interaction with Tyr-449 of S-RBD (Figure 5-2G).

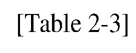

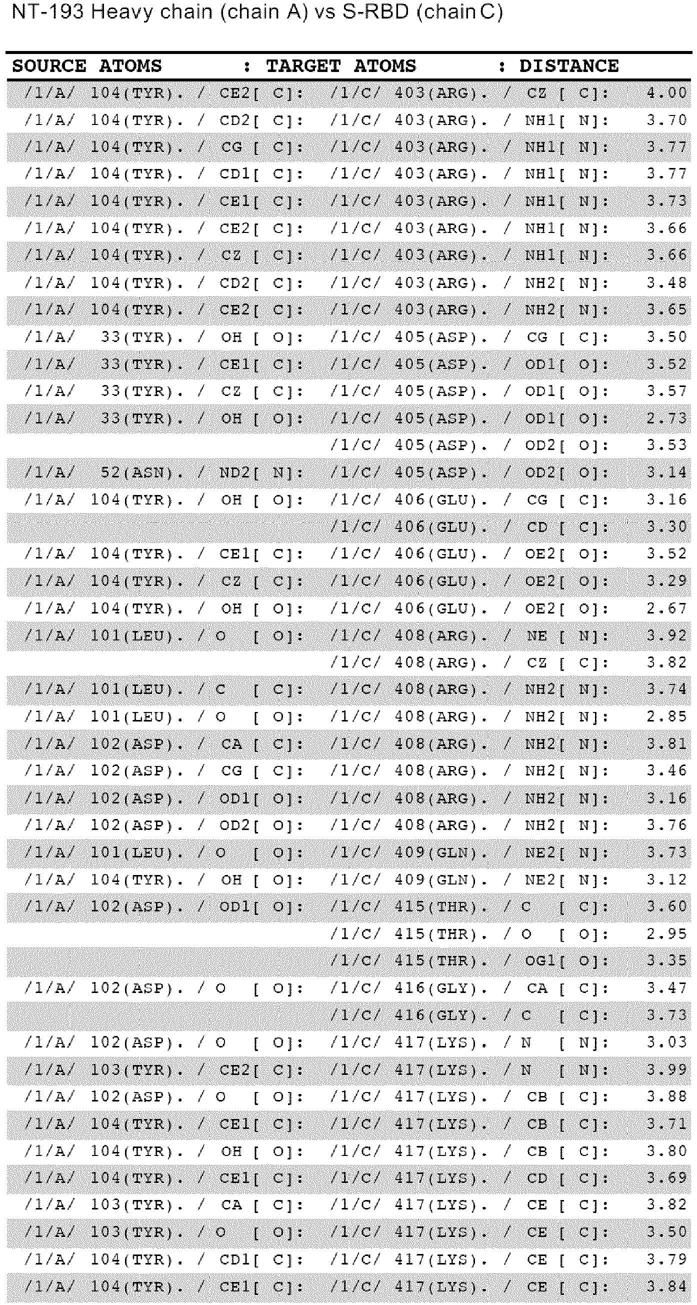

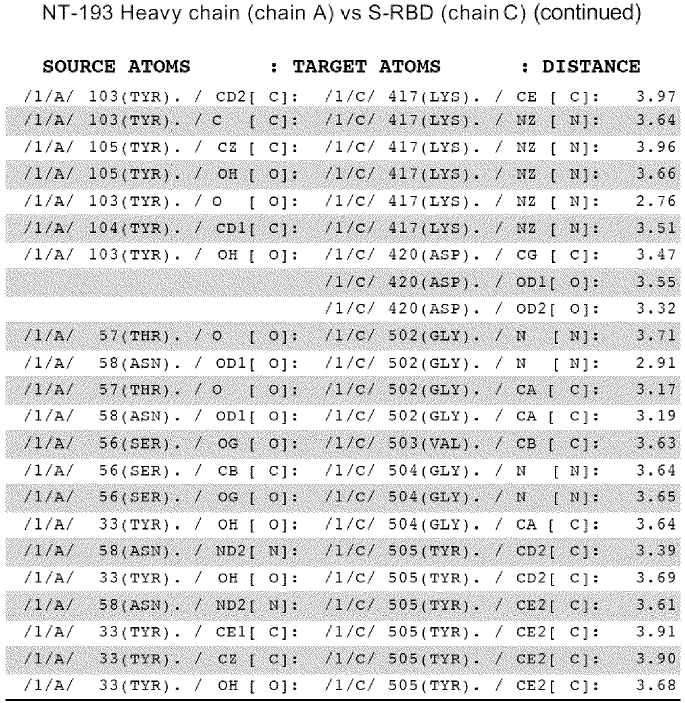

Further, the Tyr505 of S-RBD is surrounded by a hydrophobic patch comprising the Trp97 and Leu95 of CDR-L3 and the Tyr33 of CDR-H1 (Figure 6-8B). On the other hand, the Tyr33 of CDR-L1 performs a hydrophobic interaction with Leu455, and performs a polar interaction with Lys417 and Tyr453 (Figure 5-2H). Interestingly, the main chain of a DE loop binds to S-RBD via a hydrogen bond, and is also sandwiched between Tyr-489 and Phe-486 (Figures 5-2C and 5-2I). Subsequently, in heavy chain CDR-H3, a large hydrophobic interaction is observed, and three consecutive tyrosine residues that recognize a CoV-1/2 preserved region, namely, Tyr-103, Tyr-104 and Tyr-105(Figure 5-2F) are comprised therein. In particular, Tyr-104 is deeply protruded, and performs a π-cation interaction with the Lys-417 and Arg-403 of S-RBD (Figure 6-8C). As mentioned above, the epitope of the light chain of NT-193 on CoV2 S-RBD covers a major part of the ACE2-binding site, whereas the CDR-H3 of the heavy chain thereof recognizes the CoV-1/2 preserved region that would be associated with the formation of a trimer before membrane fusion (Figure 6-8A). In contrast to the heavy chains of other reported antibodies, NT-193 selectively targets a vulnerable site with a minimally necessary footprint, and achieves both strong neutralizing activity and cross-reactivity (Figure 6-9).

In recent years, several variant strains have appeared, and the pathogenicity, contagiosity, and antigenicity of the variant strains, which are called "variants of concern (VOCs)," are potentially replaced. Next, the cross-neutralizing activity of NT-193 to several VOC strains (three strains from 501Y.V1 and 1 strain from 501Y.V3) was evaluated (Figure 5-3A). It should be noted that the 501Y.V3 strain has E484K mutation that reduces the neutralizing activity of RBS antibodies including REGN10933 and LY-CoV555 by 10 to 1000 times, when it is used in monotherapy (Reference 25). Moreover, the 501Y.V3 strain has a mutation of bear K417 that enables escape from REGN10933 and LY-COV016 (References 4, 26, and 27). Regardless of these mutations, NT-193 neutralized all of these VOCs, even though it was used at a lower concentration than needed for neutralizing the original Wuhan strain (data are replaced with IC50 before submission). These data emphasize an increase in the resistance of NT-193 to the appearance of escape mutation from conventional RBS antibodies.

Using Syrian hamster models, the prophylactic and therapeutic efficacy of an NT-193 monotherapy or an NT-108/NT-193 cocktail therapy was examined. This cocktail prevented the appearance of an escape mutant under *in vitro* escape screening (Figure 6-8). Two hours before (prophylactic) or one day after (therapeutic) intranasal (therapeutic) challenge inoculation of SARS-CoV-2 to hamsters, NT-193 or an NT-108/NT-193 cocktail was administered at two types of doses (5 mg/kg and 1.25 mg/kg) to the hamsters (Figure 5-4A). With regard to the prophylaxis by the single administration or the cocktail administration, a severe reduction in the body weight of the hamsters was prevented until Day 6 after the infection (Figure 5-4B). With regard to the prophylactic effects thereof, the 5 mg/kg antibody was more significant than the 1.25 mg/kg antibody, and the effects became clear in the early stage that was on Day 2 after the infection. A difference in the body weight between a treatment group and a non-treatment group was further increased at a later time point. As with the prophylactic treatment, all of the therapeutic treatments prevented a reduction in the body weight of the hamsters until Day 6 after the infection (Figure 5-4C), but the therapeutic effects were slightly attenuated, compared with the prophylactic effects. Furthermore, prophylactic effects and therapeutic effects on the pathologic conditions of the lung caused by SARS-CoV-2 were also evaluated. On Day 6, regardless of a single administration or a cocktail administration, the lung of the antibody-administered hamsters had less inflammatory damage than the lung of control hamsters (Figure 5-4D and Figure 6-9).

The clear characteristic of the binding mode of NT-193 is bimodal antigen recognition that is unique among virus-neutralizing antibodies. Cross-reactivity is mainly acquired by heavy chain recognition of a preserved site, and neutralization is achieved by light chain recognition of RBS (Figure 5-2C and Figure 6-7B). Important contact sites that enable high affinity bond by NT-193 are R403 and K417 in a CoV/CoV2 preserved site. Non-preservability of these amino acids is probably caused by the lack of NT-193 cross-reactivity to MERS (P403 and P417) (Figures 5-2D and 5-2E, and Figure 6-9). In contrast, the relative preservability of these amino acids in viruses of clade 1b (SARS-CoV-2, etc.), clade 1a (SARS-CoV, etc.), clade 2 HKU3, and clade 3 (BM48-31, etc.) means that NT-193 broadly covers these SARS-like viruses in a plurality of clades (Reference 28).

The neutralizing RBS antibody is often encoded by public V_{H} genes that share similar binding mode and footprint with one another (References 9 and 10). Since such a concentrated antibody response involves limited diversity, it can increase the risk of generating antibody escape mutation. As a matter of fact, persistent infection in COVID-19 patients who have fallen in an immunocompromised state generates several variants including those having an E484K mutation (Reference 29), and it enabled escape from convergent neutralizing antibodies (Reference 30). Emerging VOCs have an E484K mutation (Reference 31), and cause concerns about the escape of viruses from vaccine-induced immunization and therapeutic antibodies. Hence, it is necessary to take measures that have resistance to such escape mutation. Further, it is desired to increase a wide range of neutralizing activity against SARS-like CoVs that circulate in an animal reservoir, which could cause pandemic. The present inventors consider that NT-193 is one candidate having such activity via a unique binding mode. Further structural information regarding the binding mode can lead a wide range of vaccines and therapeutic drugs to a plurality of SARS-like CoVs and appearing escape mutants.

### (4) References

Details of References 1 to 33 described in the present examples are shown below.
Reference 1. M. I. J. Raybould, A. Kovaltsuk, C. Marks, C. M. Deane, CoV-AbDab: the Coronavirus Antibody Database. Bioinformatics, (2020).
Reference 2. M. Yuan, H. Liu, N. C. Wu, I. A. Wilson, Recognition of the SARS-CoV-2 receptor binding domain by neutralizing antibodies. Biochem Biophys Res Commun, (2020).
Reference 3. L. Piccoli et al., Mapping Neutralizing and Immunodominant Sites on the SARS-CoV-2 Spike Receptor-Binding Domain by Structure-Guided High-Resolution Serology. Cell 183, 1024-1042.e1021 (2020).
Reference 4. A. Baum et al., Antibody cocktail to SARS-CoV-2 spike protein prevents rapid mutational escape seen with individual antibodies. Science 369, 1014-1018 (2020).
Reference 5. Q. Li et al., The Impact of Mutations in SARS-CoV-2 Spike on Viral Infectivity and Antigenicity. Cell 182, 1284-1294.e1289 (2020).
Reference 6. Y. Weisblum et al., Escape from neutralizing antibodies by SARS-CoV-2 spike protein variants. Elife 9, (2020).
Reference 7. H. A. Satofuka, S.; Moriwaki, T.; Okada, A.; Kazuki, K.; Tanaka, H.; Yamazaki, K.; Hichiwa, G.; Morimoto, K.; Takayama, H.; Nakayama, Y.; Hatano, S.; Baba, Y.; Oshimura, M.; Tomizuka, K.; Kazuki, Y., Efficient human-like antibody repertoire and hybridoma production in trans-chromosomic mice carrying megabase-sized human immunoglobulin loci. Preprint (version 1) available at Research Square, (2020).
Reference 8. Y. Adachi et al., Exposure of an occluded hemagglutinin epitope drives selection of a class of cross-protective influenza antibodies. Nat Commun 10, 3883 (2019).
Reference 9. D. F. Robbiani et al., Convergent antibody responses to SARS-CoV-2 in convalescent individuals. Nature 584, 437-442 (2020).
Reference 10. M. Yuan et al., Structural basis of a shared antibody response to SARS-CoV-2. Science 369, 1119-1123 (2020).
Reference 11. C. M. Tipton, J. R. Hom, C. F. Fucile, A. F. Rosenberg, I. Sanz, Understanding B-cell activation and autoantibody repertoire selection in systemic lupus erythematosus: A B-cell immunomics approach. Immunol Rev 284, 120-131 (2018). Reference 12. M. C. Woodruff et al., Extrafollicular B cell responses correlate with neutralizing antibodies and morbidity in COVID-19. Nat Immunol 21, 1506-1516 (2020).
Reference 13. S. J. Zost et al., Rapid isolation and profiling of a diverse panel of human monoclonal antibodies targeting the SARS-CoV-2 spike protein. Nat Med 26, 1422-1427 (2020).
Reference 14. C. O. Barnes et al., SARS-CoV-2 neutralizing antibody structures inform therapeutic strategies. Nature 588, 682-687 (2020).
Reference 15. M. Yuan et al., A highly preserved cryptic epitope in the receptor binding domains of SARS-CoV-2 and SARS-CoV. Science 368, 630-633 (2020).
Reference 16. A. Baum et al., REGN-COV2 antibodies prevent and treat SARS-CoV-2 infection in rhesus macaques and hamsters. Science 370, 1110-1115 (2020).
Reference 17. D. M. Weinreich et al., REGN-COV2, a Neutralizing Antibody Cocktail, in Outpatients with Covid-19. N Engl J Med, (2020).
Reference 18. D. Pinto et al., Cross-neutralization of SARS-CoV-2 by a human monoclonal SARS-CoV antibody. Nature 583, 290-295 (2020).
Reference 19. J. Hansen et al., Studies in humanized mice and convalescent humans yield a SARS-CoV-2 antibody cocktail. Science 369, 1010-1014 (2020).
Reference 20. P. J. M. Brouwer et al., Potent neutralizing antibodies from COVID-19 patients define multiple targets of vulnerability. Science 369, 643-650 (2020). Reference 21. Z. Ke et al., Structures and distributions of SARS-CoV-2 spike proteins on intact virions. Nature 588, 498-502 (2020).
Reference 22. R. P. Galimidi et al., Intra-spike crosslinking overcomes antibody evasion by HIV-1. Cell 160, 433-446 (2015).
Reference 23. O. Scharf et al., Immunoglobulin G3 from polyclonal human immunodeficiency virus (HIV) immune globulin is more potent than other subclasses in neutralizing HIV type 1. J Virol 75, 6558-6565 (2001).
Reference 24. M. Kobayashi et al., Selection of diverse and clinically relevant integrase inhibitor-resistant human immunodeficiency virus type 1 mutants. Antiviral Res 80, 213-222 (2008).
Reference 25. P. Wang et al., Increased Resistance of SARS-CoV-2 Variants B.1.351 and B.1.1.7 to Antibody Neutralization. bioRxiv, 2021.2001.2025.428137 (2021).
Reference 26. R. Shi et al., A human neutralizing antibody targets the receptor-binding site of SARS-CoV-2. Nature 584, 120-124 (2020).
Reference 27. T. N. Starr et al., Prospective mapping of viral mutations that escape antibodies used to treat COVID-19. Science, (2021).
Reference 28. C. G. Rappazzo et al., Broad and potent activity against SARS-like viruses by an engineered human monoclonal antibody. Science, (2021).
Reference 29. B. Choi et al., Persistence and Evolution of SARS-CoV-2 in an Immunocompromised Host. N Engl J Med 383, 2291-2293 (2020).
Reference 30. C. Gaebler et al., Evolution of Antibody Immunity to SARS-CoV-2. bioRxiv, (2020).
Reference 31. H. Tegally et al., Emergence and rapid spread of a new severe acute respiratory syndrome-related coronavirus 2 (SARS-CoV-2) lineage with multiple spike mutations in South Africa. medRxiv, 2020.2012.2021.20248640 (2020).
Reference 32. F. Amanat et al., A serological assay to detect SARS-CoV-2 seroconversion in humans. Nat Med 26, 1033-1036 (2020).
Reference 33. T. Tiller et al., Efficient generation of monoclonal antibodies from single human B cells by single cell RT-PCR and expression vector cloning. Journal of immunological methods 329, 112-124 (2008).

### Sequence Listing Free Text

SEQ ID NO: 21: Synthetic DNA
SEQ ID NO: 24: Synthetic peptide
SEQ ID NO: 31: Synthetic DNA
SEQ ID NO: 32: Synthetic DNA
SEQ ID NO: 33: Synthetic DNA

## Claims

1. An antibody against a spike protein of coronavirus.

2. The antibody according to claim 1, wherein the spike protein is a protein in an extracellular region or a receptor-binding domain.

3. The antibody according to claim 1 or 2, wherein the epitope of the spike protein is composed of at least two amino acid residues selected from the group consisting of R403, D405, E406, R408, T415, G416, K417, D420, Y449, Y453, L455, F456, G485, Y486, N487, Y489, Q493, S494, Y495, G496, Q498, N501, G502, V503, G504 and Y505, in the full-length amino acid sequence of the receptor-binding domain.

4. The antibody according to claim 3, wherein the two amino acid residues are R403 and K417.

5. The antibody according to claim 1, wherein the coronavirus is SARS-CoV1, SARS-CoV2, or MERS-CoV.

6. The antibody according to any one of claims 1 to 5, which has a neutralizing activity of less than 1 ng/ml as an IC50 value that is an indicator of suppression of viral proliferation.

7. The antibody according to any one of claims 1 to 6, wherein the amino acid sequence of a heavy chain variable region thereof is as set forth in SEQ ID NO: 14 or 18.

8. The antibody according to any one of claims 1 to 6, wherein the amino acid sequence of a light chain variable region thereof is as set forth in SEQ ID NO: 16 or 20.

9. An antibody that binds to an epitope, to which the antibody according to any one of claims 1 to 8 binds.

10. The antibody according to any one of claims 1 to 9, which is a chimeric antibody, a humanized antibody, or a human antibody.

11. A pharmaceutical composition comprising the antibody according to any one of claims 1 to 10.

12. The pharmaceutical composition according to claim 11, which is for use in the treatment of coronavirus disease.

13. A reagent for detecting coronavirus, comprising the antibody according to any one of claims 1 to 10.

14. A method for detecting coronavirus, comprising a step of allowing the antibody according to any one of claims 1 to 10 to come into contact with a collected biological sample.
